# EUROPEAN PATENT APPLICATION

(11) **EP 4 506 334 A1**
(43) Date of publication of application: **12.02.2025**
(21) Application number: 23799482.7
(22) Date of filing: 28.04.2023
(51) Int. Cl.: C07C 303/28, C07C 303/36, C07C 309/66, C07C 311/08

(54) **TRIFLUOROMETHANE SULFONYLATION AGENT COMPOSITION AND METHOD FOR PRODUCING TRIFLUOROMETHANESULFONYLOXY COMPOUND OR TRIFLUOROMETHANE SULFONYL COMPOUND**

(30) Priority: 02.05.2022 JP 2022076302
(71) Applicant: Central Glass Company, Limited, Ube-shi, Yamaguchi 755-0001 (JP)
(72) Inventor: TOMITA Ren, Tokyo 101-0054 (JP); NITTA Junki, Tokyo 101-0054 (JP); YAMAZAKI Takako, Tokyo 101-0054 (JP)
(74) Representative: Manitz Finsterwald Patent- und Rechtsanwaltspartnerschaft mbB
(86) International application number: PCT/JP2023/016923
(87) International publication number: WO 2023/214552

(57) **Abstract**

The present disclosure provides a trifluoromethanesulfonylating agent composition containing a compound represented by Formula (1) or (11) described in the specification, and a method for producing a trifluoromethanesulfonyloxy compound or a method for producing a trifluoromethanesulfonyl compound, which includes reacting the trifluoromethanesulfonylating agent composition with a compound represented by Formula (2) described in the specification, or a specific substrate.

## Description

### TECHNICAL FIELD

The present disclosure relates to a trifluoromethanesulfonylating agent composition capable of trifluoromethanesulfonylating a substrate containing a functional group such as a phenolic hydroxyl group, and a method for producing a trifluoromethanesulfonyloxy compound or a trifluoromethanesulfonyl compound using the same.

### BACKGROUND ART

In a synthesis of active pharmaceutical ingredients or pharmaceutical intermediates, trifluoromethanesulfonylation of substrates having various functional groups is carried out.

The trifluoromethanesulfonylation of the phenolic hydroxyl group is an important reaction in the synthesis of the active pharmaceutical ingredients or pharmaceutical intermediates. Examples thereof include a method in which a substrate having a phenolic hydroxyl group is allowed to react with trifluoromethanesulfonic anhydride (Patent Literature 1, Non-Patent Literature 1, and Non-Patent Literature 2), a method in which a substrate having a phenolic hydroxyl group is allowed to react with trifluoromethanesulfonyl fluoride (Patent Literature 2), a method in which a substrate having a phenolic hydroxyl group is allowed to react with trifluoromethanesulfonyl chloride (Patent Literature 3), a method in which a substrate having a phenolic hydroxyl group is allowed to react with N-phenyl triflimide (Patent Literature 4), a method in which a substrate having a phenolic hydroxyl group is allowed to react with 5-chloro-2-pyridyl triflimide (Patent Literature 5), a method in which a substrate having a phenolic hydroxyl group is allowed to react with 4-nitrophenyl trifluoromethanesulfonate (Non-Patent Literature 3), and a method in which a substrate having a phenolic hydroxyl group is allowed to react with 2-pyridyl triflimide (Patent Literature 6).

In addition to the substrate having a phenolic hydroxyl group, ketones, primary amines, or secondary amines have also been trifluoromethanesulfonylated.

As a trifluoromethanesulfonylation reaction, for example, Non-Patent Literatures 4 and 5 disclose a method in which a substrate having a metalloenolate, a phenolic hydroxyl group, or a secondary amine is allowed to react with N-phenyl triflimide.

Further, Non-Patent Literature 6 discloses a method in which a metalloenolate is allowed to react with N-(2-pyridyl) triflimide, or a metalloenolate is allowed to react with N-5-chloro-2-pyridyl triflimide. Non-Patent Literatures 7 and 8 disclose a method in which a ketone is allowed to react with trifluoromethanesulfonic anhydride.

### CITATION LIST

### PATENT LITERATURE

Patent Literature 1: CN104230960A
Patent Literature 2: JP2002-128752A
Patent Literature 3: WO2011/095625A
Patent Literature 4: WO2014/190271A
Patent Literature 5: WO2013/044092A
Patent Literature 6: WO2007/073503A

### NON-PATENT LITERATURE

Non Patent Literature 1: P.Kancharla. et al, Journal of Medicial Chemistry, 2020, 63, 6179-6202.
Non Patent Literature 2: D.Xi.et al, European Journal of Medicinal Chemistry. 2019, 178, 802-817.
Non Patent Literature 3: M.Lesperance. et al, Steroids. 2018, 140, 104-113
Non Patent Literature 4: J.B; Hendrickson. et al, Tetrahedron Letters, 1973, 46, 4607-4610.
Non Patent Literature 5: J.E.McMurry. et al, Tetrahedron Letters, 1983, 10, 979-982.
Non Patent Literature 6: d.L.Comins. et al, Tetrahedron Letters, 1992, 42, 6299-6302.
Non Patent Literature 7: M.Tranchant. et al, Tetrahedron, 2002, 58, 8425-8432.
Non Patent Literature 8: P.J.Stang. et al, Synthesis. 1980, 4, 283-284.

### SUMMARY OF INVENTION

### TECHNICAL PROBLEM

However, there is still room for further study of trifluoromethanesulfonylation reagents from the viewpoints of economics and reduction of by-products.

The present disclosure has been made in consideration of these circumstances. An object of the present disclosure is to provide a trifluoromethanesulfonylating agent composition capable of trifluoromethanesulfonylating a substrate having a functional group such as a phenolic hydroxyl group. Another object of the present disclosure is to provide an efficiently and industrially feasible method for producing a trifluoromethanesulfonyloxy compound or a trifluoromethanesulfonyl compound.

### SOLUTION TO PROBLEM

Therefore, in view of the above problems, the present inventors have conducted extensive research. As a result, the inventors have found that the trifluoromethanesulfonylating agent composition of the present disclosure, which contains a specific trifluoromethanesulfonylating agent, can trifluoromethanesulfonylate a substrate having a functional group such as a phenolic hydroxyl group with preventing production of by-products. Further, the inventors have found that a trifluoromethanesulfonyloxy compound can be isolated from a reaction solution after a trifluoromethanesulfonylation reaction by merely carrying out a general post-treatment operation, and a trifluoromethanesulfonyloxy compound or a trifluoromethanesulfonyl compound can be efficiently obtained.

That is, the present disclosure provides inventions described in the following [1] to [16].
[1] A trifluoromethanesulfonylating agent composition containing:
   a compound represented by the following Formula (1) or (11): 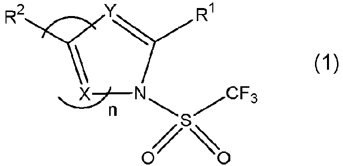 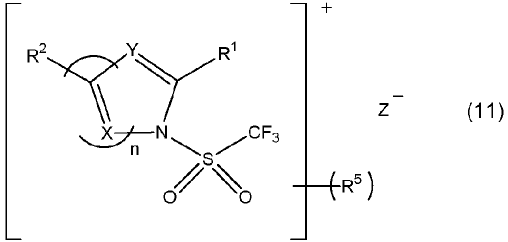
   in which in Formulae (1) and (11), R¹ is a hydrogen atom, a linear alkyl group having 1 to 6 carbon atoms, or a branched alkyl group having 3 to 6 carbon atoms,
   R² is a hydrogen atom, a linear aliphatic hydrocarbon group having 1 to 6 carbon atoms, a branched aliphatic hydrocarbon group having 3 to 6 carbon atoms, a nitro group, an aromatic hydrocarbon group having 6 to 14 carbon atoms, or an aromatic heterocyclic group having 6 to 14 carbon atoms,
   X is a nitrogen atom or C(R³),
   Y is a nitrogen atom or C(R⁴),
   R³ is a hydrogen atom, a linear aliphatic hydrocarbon group having 1 to 6 carbon atoms, a branched aliphatic hydrocarbon group having 3 to 6 carbon atoms, an aromatic hydrocarbon group having 6 to 14 carbon atoms, or an aromatic heterocyclic group having 6 to 14 carbon atoms,
   R⁴ is a hydrogen atom, a linear aliphatic hydrocarbon group having 1 to 6 carbon atoms, a branched aliphatic hydrocarbon group having 3 to 6 carbon atoms, an aromatic hydrocarbon group having 6 to 14 carbon atoms, or an aromatic heterocyclic group having 6 to 14 carbon atoms,
   R² and R³ may be bonded to form a ring, and R² and R⁴ may be bonded to form a ring, and
   n is an integer of 1 to 3;
   when there are a plurality of R², R² may be the same or different, and when there are a plurality of R³, R³ may be the same or different; and
   in Formula (11), R⁵ is a hydrogen atom, a linear alkyl group having 1 to 6 carbon atoms, or a branched alkyl group having 3 to 6 carbon atoms, and
   Z⁻ is an anion.
[2] The trifluoromethanesulfonylating agent composition according to [1], further including:
   a compound represented by Formula (1); and
   any base selected from the group consisting of an aliphatic organic base, an organic base having a heterocyclic group, and an inorganic base.
[3] The trifluoromethanesulfonylating agent composition according to [2],
   in which in Formula (1), X is C(R³), and R¹, R², and R³ each independently represent a hydrogen atom, a methyl group, an ethyl group, an n-propyl group, or an isopropyl group.
[4] The trifluoromethanesulfonylating agent composition according to [2] or [3],
   in which the organic base is selected from the group consisting of a secondary amine, a tertiary amine, an alkoxide, and an organic base having a heterocyclic group having a nitrogen atom and 4 or more carbon atoms.
[5] The trifluoromethanesulfonylating agent composition according to any one of [2] to [4], in which the inorganic base is selected from the group consisting of a potassium salt and a sodium salt.
[6] The trifluoromethanesulfonylating agent composition according to any one of [2] to [5],
   in which the base is any one selected from the group consisting of sodium carbonate, potassium carbonate, sodium bicarbonate, potassium bicarbonate, lithium methoxide, lithium ethoxide, lithium tert-butoxide, sodium methoxide, sodium ethoxide, sodium tert-butoxide, potassium methoxide, potassium ethoxide, potassium tert-butoxide, triethylamine, tri-n-propylamine, tributylamine, diisopropylethylamine, N,N-diethylcyclohexylamine, 1,8-diazabicyclo[5.4.0]undecene, and 1,5-diazabicyclo[4.3.0]nonene.
[7] A method for producing a trifluoromethanesulfonyloxy compound, including:
   allowing the trifluoromethanesulfonylating agent composition according to any one of [2] to [6] to react with a hydroxylated aromatic compound represented by Formula (2):

      **Ar-OH** (2)
   in which in Formula (2), Ar represents an aromatic ring group or a substituted aromatic ring group.
[8] The method for producing a trifluoromethanesulfonyloxy compound according to [7],
   in which in Formula (2), Ar represents an aromatic ring group, and the aromatic ring group is an aromatic heterocyclic group.
[9] The method for producing a trifluoromethanesulfonyloxy compound according to [7],
   in which in Formula (2), Ar represents a substituted aromatic ring group, and a substituent of the substituted aromatic ring group is a lower alkyl group, a lower alkoxycarbonyl lower alkyl group, a β-D-glucopyranoside group, an amino group, a lower alkylamino group, or a hydroxyl group.
[10] The method for producing a trifluoromethanesulfonyloxy compound according any one of [7] to [9],
   in which the reaction is performed at a reaction temperature of 150°C or lower.
[11] The method for producing a trifluoromethanesulfonyloxy compound according to any one of [7] to [10],
   in which after completion of the reaction, a reaction solution is post-treated with an acidic aqueous solution.
[12] The trifluoromethanesulfonylating agent composition according to [1],
   in which a compound represented by Formula (11) is a compound represented by the following Formula (11a): 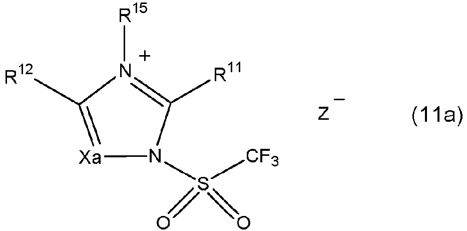
   in which in Formula (11a), R¹¹, R¹², and R¹⁵ are a hydrogen atom, a linear alkyl group having 1 to 6 carbon atoms, or a branched alkyl group having 3 to 6 carbon atoms,
   Xa is a nitrogen atom or C(R¹³),
   R¹³ is a hydrogen atom, a linear alkyl group having 1 to 6 carbon atoms, or a branched alkyl group having 3 to 6 carbon atoms, and
   R¹² and R¹³ may be bonded to form a ring; and
   Z⁻ is an anion.
[13] The trifluoromethanesulfonylating agent composition according to [12],
   in which Z⁻ in Formula (11a) is a trifluoromethanesulfonate anion, a fluoromethanesulfonate anion, a methanesulfonate anion, or a sulfonate anion.
[14] The trifluoromethanesulfonylating agent composition according to [12] or [13],
   in which Xa in Formula (11a) is C(R¹³).
[15] The trifluoromethanesulfonylating agent composition according to any one of [12] to [14],
   in which in Formula (11a), Xa is C(R¹³), and R¹¹, R¹², R¹³, and R¹⁵ are each independently a hydrogen atom, a methyl group, an ethyl group, an n-propyl group, or an isopropyl group.
[16] A method for producing a trifluoromethanesulfonyl compound, including:
   allowing the trifluoromethanesulfonylating agent composition according to any one of [12] to [15] to react with at least one substrate selected from the group consisting of a compound having a phenolic hydroxyl group, a compound having an alcoholic hydroxyl group, a ketone, a primary amine, and a secondary amine.

### EFFECTS OF INVENTION

According to the present disclosure, it is possible to provide a trifluoromethanesulfonylating agent composition capable of trifluoromethanesulfonylating a substrate having a functional group such as a phenolic hydroxyl group. Further, it is possible to provide an industrially feasible and efficient method for producing trifluoromethanesulfonyloxy compound or trifluoromethanesulfonyl compound using the above-described trifluoromethanesulfonylating agent composition.

### DESCRIPTION OF EMBODIMENTS

Hereinafter, the present disclosure will be described in detail. Hereinafter, embodiments of the present disclosure will be described, but the present disclosure is not limited to the following embodiments, and can be implemented appropriately based on ordinary knowledge of those skilled in the art, as long as the spirit of the present disclosure is not impaired.

### <Trifluoromethanesulfonylating Agent Composition>

### (Trifluoromethanesulfonylating Agent)

A trifluoromethanesulfonylating agent composition of the present disclosure (hereinafter also referred to as a composition of the present disclosure) contains a compound represented by the following Formula (1) or (11) as a trifluoromethanesulfonylating agent.

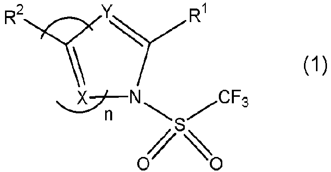

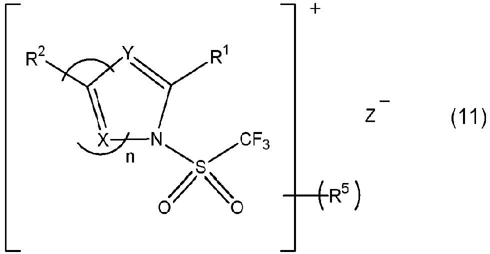

In Formulae (1) and (11), R¹ is a hydrogen atom, a linear alkyl group having 1 to 6 carbon atoms, or a branched alkyl group having 3 to 6 carbon atoms,
R² is a hydrogen atom, a linear aliphatic hydrocarbon group having 1 to 6 carbon atoms, a branched aliphatic hydrocarbon group having 3 to 6 carbon atoms, a nitro group, an aromatic hydrocarbon group having 6 to 14 carbon atoms, or an aromatic heterocyclic group having 6 to 14 carbon atoms,
X is a nitrogen atom or C(R³), and Y is a nitrogen atom or C(R⁴),
R³ is a hydrogen atom, a linear aliphatic hydrocarbon group having 1 to 6 carbon atoms, a branched aliphatic hydrocarbon group having 3 to 6 carbon atoms, an aromatic hydrocarbon group having 6 to 14 carbon atoms, or an aromatic heterocyclic group having 6 to 14 carbon atoms,
R⁴ is a hydrogen atom, a linear aliphatic hydrocarbon group having 1 to 6 carbon atoms, a branched aliphatic hydrocarbon group having 3 to 6 carbon atoms, an aromatic hydrocarbon group having 6 to 14 carbon atoms, or an aromatic heterocyclic group having 6 to 14 carbon atoms,
R² and R³ may be bonded to form a ring, and R² and R⁴ may be bonded to form a ring, and
n is an integer of 1 to 3.

When there are a plurality of R², R² may be the same or different, and when there are a plurality of R³, R³ may be the same or different.

In Formula (11), R⁵ is a hydrogen atom, a linear alkyl group having 1 to 6 carbon atoms, or a branched alkyl group having 3 to 6 carbon atoms, and
Z⁻ is an anion.

In Formulae (1) and (11), R¹ represents a hydrogen atom, a linear alkyl group having 1 to 6 carbon atoms, or a branched alkyl group having 3 to 6 carbon atoms.

Examples of the linear alkyl group having 1 to 6 carbon atoms include a methyl group, an ethyl group, an n-propyl group, an n-butyl group, an n-pentyl group, and an n-hexyl group.

Examples of the branched alkyl group having 3 to 6 carbon atoms include an isopropyl group, an isobutyl group, a sec-butyl group, and a tert-butyl group.

Among these, R¹ is preferably a hydrogen atom, a methyl group, an ethyl group, an n-propyl group, or an isopropyl group, because of ease of synthesis.

In Formulae (1) and (11), R² represents a hydrogen atom, a linear aliphatic hydrocarbon group having 1 to 6 carbon atoms, a branched aliphatic hydrocarbon group having 3 to 6 carbon atoms, a nitro group, an aromatic hydrocarbon group having 6 to 14 carbon atoms, or an aromatic heterocyclic group having 6 to 14 carbon atoms. When there are a plurality of R², R² may be the same or different.

Examples of the linear aliphatic hydrocarbon group having 1 to 6 carbon atoms include a linear alkyl group having 1 to 6 carbon atoms, a linear alkenyl group having 2 to 6 carbon atoms, and a linear alkynyl group having 2 to 6 carbon atoms.

Examples of the linear alkyl group having 1 to 6 carbon atoms include the examples of the linear alkyl group having 1 to 6 carbon atoms which is described as R¹.

Examples of the branched aliphatic hydrocarbon group having 3 to 6 carbon atoms include a branched alkyl group having 3 to 6 carbon atoms, a branched alkenyl group having 3 to 6 carbon atoms, and a branched alkynyl group having 3 to 6 carbon atoms.

Examples of the branched alkyl group having 3 to 6 carbon atoms include the examples of the branched alkyl group having 3 to 6 carbon atoms which is described as R¹.

Examples of the aromatic hydrocarbon group having 6 to 14 carbon atoms include a phenyl group, a naphthyl group, and an anthryl group.

Examples of the aromatic heterocyclic group having 6 to 14 carbon atoms include a pyrrole group, a pyrazine group, a pyrimidine group, and a pyridazine group.

R² is preferably a hydrogen atom, a linear alkyl group having 1 to 6 carbon atoms, a branched alkyl group having 3 to 6 carbon atoms, or a nitro group, and is more preferably a hydrogen atom, a linear alkyl group having 1 to 6 carbon atoms, or a branched alkyl group having 3 to 6 carbon atoms.

R² is preferably a hydrogen atom, a methyl group, an ethyl group, an n-propyl group, an isopropyl group, or a nitro group, and more preferably a hydrogen atom, a methyl group, an ethyl group, an n-propyl group, or an isopropyl group.

In Formulae (1) and (11), X is a nitrogen atom or C(R³), and Y is a nitrogen atom or C(R⁴).

R³ represents a hydrogen atom, a linear aliphatic hydrocarbon group having 1 to 6 carbon atoms, a branched aliphatic hydrocarbon group having 3 to 6 carbon atoms, an aromatic hydrocarbon group having 6 to 14 carbon atoms, or an aromatic heterocyclic group having 6 to 14 carbon atoms. When there are a plurality of R³, R³ may be the same or different.

Examples of the linear aliphatic hydrocarbon group having 1 to 6 carbon atoms, the branched aliphatic hydrocarbon group having 3 to 6 carbon atoms, the aromatic hydrocarbon group having 6 to 14 carbon atoms, and the aromatic heterocyclic group having 6 to 14 carbon atoms include the examples of the linear aliphatic hydrocarbon group having 1 to 6 carbon atoms, the branched aliphatic hydrocarbon group having 3 to 6 carbon atoms, the aromatic hydrocarbon group having 6 to 14 carbon atoms, and the aromatic heterocyclic group having 6 to 14 carbon atoms, which are described as R².

R³ is preferably a hydrogen atom, a linear alkyl group having 1 to 6 carbon atoms, or a branched alkyl group having 3 to 6 carbon atoms, and more preferably a hydrogen atom, a methyl group, an ethyl group, an n-propyl group, or an isopropyl group.

R⁴ represents a hydrogen atom, a linear aliphatic hydrocarbon group having 1 to 6 carbon atoms, a branched aliphatic hydrocarbon group having 3 to 6 carbon atoms, an aromatic hydrocarbon group having 6 to 14 carbon atoms, or an aromatic heterocyclic group having 6 to 14 carbon atoms. When there are a plurality of R⁴, R⁴ may be the same or different.

Examples of the linear aliphatic hydrocarbon group having 1 to 6 carbon atoms, the branched aliphatic hydrocarbon group having 3 to 6 carbon atoms, the aromatic hydrocarbon group having 6 to 14 carbon atoms, and the aromatic heterocyclic group having 6 to 14 carbon atoms include the examples of the linear aliphatic hydrocarbon group having 1 to 6 carbon atoms, the branched aliphatic hydrocarbon group having 3 to 6 carbon atoms, the aromatic hydrocarbon group having 6 to 14 carbon atoms, and the aromatic heterocyclic group having 6 to 14 carbon atoms, which are described as R².

R⁴ is preferably a hydrogen atom, a linear alkyl group having 1 to 6 carbon atoms, or a branched alkyl group having 3 to 6 carbon atoms, and more preferably a hydrogen atom, a methyl group, an ethyl group, an n-propyl group, or an isopropyl group.

In Formulae (1) and (11), X is preferably C(R³), and Y is preferably a nitrogen atom.

n is an integer of 1 to 3. n is preferably 1.

R² and R³ may bonded to form a ring. The ring formed by bonding of R² and R³ is not particularly limited, and examples thereof include an aromatic hydrocarbon ring (for example, a naphthalene ring and an anthracene ring) having 6 to 14 carbon atoms or an aromatic heterocyclic ring having 6 to 14 carbon atoms.

R² and R⁴ may bonded to form a ring. The ring formed by bonding of R² and R⁴ is not particularly limited, and examples thereof include an aromatic hydrocarbon ring (for example, a naphthalene ring and an anthracene ring) having 6 to 14 carbon atoms or an aromatic heterocyclic ring having 6 to 14 carbon atoms.

In Formula (1), X is C(R³), and it is preferable that R¹, R², and R³ each independently represent a hydrogen atom, a methyl group, an ethyl group, an n-propyl group, or an isopropyl group.

In Formula (11), R⁵ is a hydrogen atom, a linear alkyl group having 1 to 6 carbon atoms, or a branched alkyl group having 3 to 6 carbon atoms.

Examples of the linear alkyl group having 1 to 6 carbon atoms and the branched alkyl group having 3 to 6 carbon atoms represented by R⁵ include the examples of the linear alkyl group having 1 to 6 carbon atoms and the branched alkyl group having 3 to 6 carbon atoms, which are described as R¹, and preferred examples are also the same.

R⁵ is preferably bonded to X or Y, and more preferably bonded to Y

In a preferred aspect, R⁵ is bonded to the nitrogen atom in X to form a cationic moiety.

Further, in a preferred aspect, R⁵ is bonded to the nitrogen atom in Y to form a cationic moiety.

In Formula (11), Z⁻ represents an anion.

Z⁻ is preferably an anion of a strong acid or a superacid, more preferably a tetrafluoroborate anion, a hexafluorophosphate anion, a hexafluoroantimonate anion, a fluorosulfonate anion, a trifluoroacetate anion, a trifluoromethanesulfonate anion, a fluoromethanesulfonate anion, a methanesulfonate anion, a toluenesulfonate anion or a sulfonate anion, more preferably a trifluoromethanesulfonate anion, a fluoromethanesulfonate anion, a methanesulfonate anion, a toluenesulfonate anion or a sulfonate anion, and still more preferably a trifluoromethanesulfonate anion.

A compound represented by Formula (11) is preferably a compound represented by the following Formula (11a).

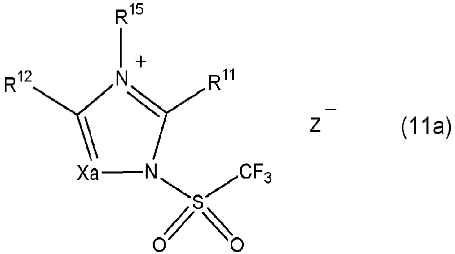

In Formula (11a), R¹¹, R¹², and R¹⁵ are a hydrogen atom, a linear alkyl group having 1 to 6 carbon atoms, or a branched alkyl group having 3 to 6 carbon atoms,
Xa is a nitrogen atom or C(R¹³),
R¹³ is a hydrogen atom, a linear alkyl group having 1 to 6 carbon atoms, or a branched alkyl group having 3 to 6 carbon atoms, and
R¹² and R¹³ may be bonded to form a ring.
Z⁻ is an anion.

Examples of the linear alkyl group having 1 to 6 carbon atoms and the branched alkyl group having 3 to 6 carbon atoms as R¹¹, R¹², and R¹⁵ include the examples of the linear alkyl group having 1 to 6 carbon atoms and the branched alkyl group having 3 to 6 carbon atoms, which are described as R¹, R², and R⁵ in Formula (11).

In Formula (11a), Xa is a nitrogen atom or C(R¹³), and preferably C(R¹³).

Examples of the linear alkyl group having 1 to 6 carbon atoms and the branched alkyl group having 3 to 6 carbon atoms as R¹³ include the examples of the linear alkyl group having 1 to 6 carbon atoms and the branched alkyl group having 3 to 6 carbon atoms, which are described as R³ in Formula (11).

In Formula (11a), it is preferable that Xa is C(R¹³), and R¹¹, R¹², R¹³, and R¹⁵ are each independently a hydrogen atom, a methyl group, an ethyl group, an n-propyl group, or an isopropyl group, and particularly preferable that Xa is a hydrogen atom or a methyl group, from the viewpoint of ease of synthesis.

Z⁻ in Formula (11a) has the same meaning as Z⁻ in Formula (11), and preferable examples thereof are also the same.

Examples of the compound represented by Formula (1) are shown below, but the compound is not limited thereto.

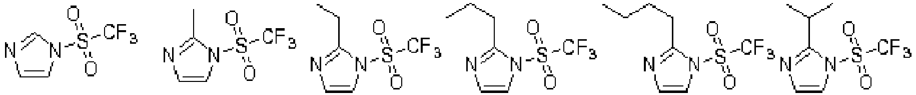

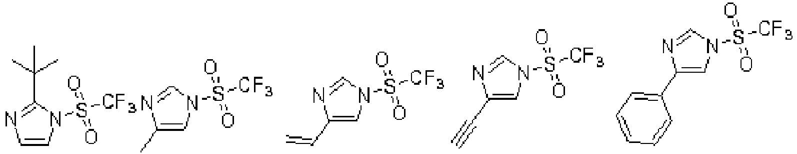

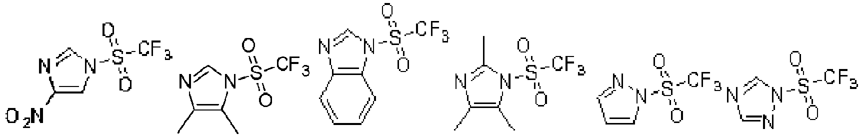

Examples of the compound represented by Formula (11) are shown below, but the compound is not limited thereto.

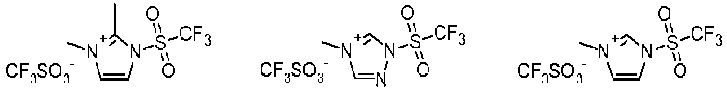

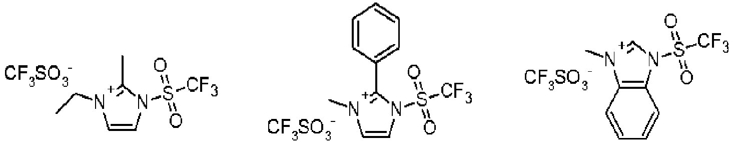

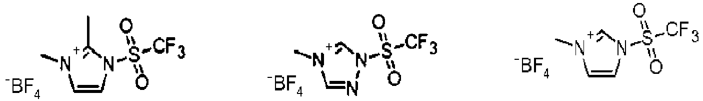

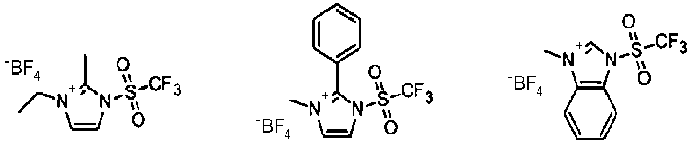

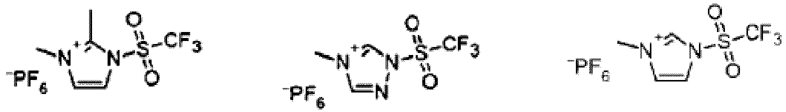

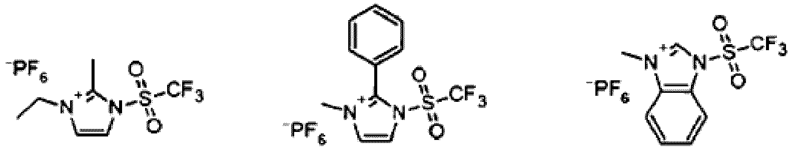

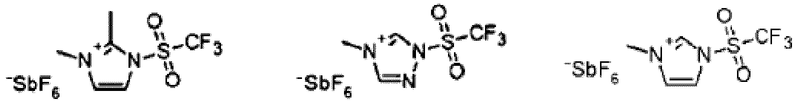

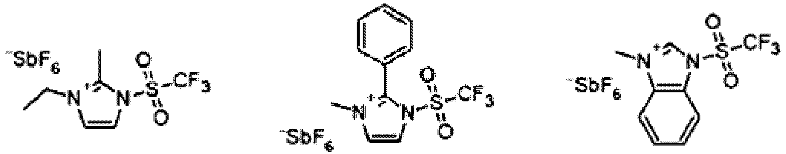

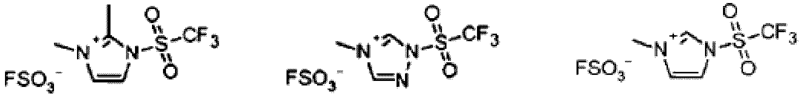

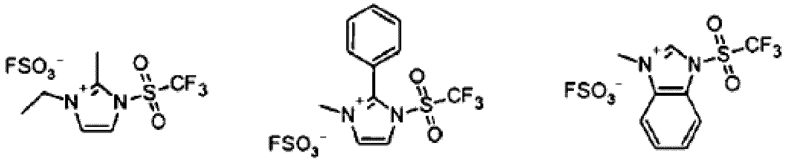

### . First Aspect

By using the trifluoromethanesulfonylating agent composition containing the compound represented by the above-mentioned Formula (1) or (11), it is possible to trifluoromethanesulfonylate a substrate having a phenolic hydroxyl group or the like, and one preferred aspect of the present disclosure is a trifluoromethanesulfonylating agent composition containing the compound represented by the above-mentioned Formula (1) and any base selected from the group consisting of an aliphatic organic base, an organic base having a heterocyclic group, and an inorganic base.

A trifluoromethanesulfonylating agent composition containing the compound represented by Formula (1) and a specific base (hereinafter also referred to as a first trifluoromethanesulfonylating agent composition, or simply as a first composition) can selectively trifluoromethanesulfonylate the phenolic hydroxyl group with preventing production of by-products. Further, the trifluoromethanesulfonyloxy compound can be isolated from a reaction solution after a trifluoromethanesulfonylation reaction by simply performing a general post-treatment operation, and a trifluoromethanesulfonyloxy compound can be obtained industrially and efficiently.

In this specification, the meaning that the trifluoromethanesulfonylating agent "selectively reacts" with a phenolic hydroxyl group is that the trifluoromethanesulfonylation reaction proceeds preferentially with the phenolic hydroxyl group.

The first composition preferably contains the compound represented by Formula (1) in an amount of 80% or more, and more preferably 90% or more, based on a total mass of the first composition.

### (Base)

The first trifluoromethanesulfonylating agent composition according to the present disclosure preferably contains a base.

Examples of the base used in the first trifluoromethanesulfonylating agent composition include any base selected from the group consisting of an aliphatic organic base, an organic base having a heterocyclic group, and an inorganic base.

The organic base is preferably a base selected from the group consisting of a secondary amine, a tertiary amine, an alkoxide, and an organic base having a heterocyclic group having a nitrogen atom and 4 or more carbon atoms.

The aliphatic organic base is an organic base having an aliphatic hydrocarbon group and having no aromatic group.

Specifically, a secondary amine, a tertiary amine, or an alkoxide are preferred, and examples thereof include lithium methoxide, lithium ethoxide, lithium tert-butoxide, sodium methoxide, sodium ethoxide, sodium tert-butoxide, potassium methoxide, potassium ethoxide, potassium tert-butoxide, dipropylamine, diisobutylamine, trimethylamine, triethylamine, tri-n-propylamine, tributylamine, diisopropylethylamine, and N,N-diethylcyclohexylamine.

The organic base having a heterocyclic group is preferably a compound having a heterocyclic group having 4 or more carbon atoms, and more preferably a compound having a heterocyclic group having a nitrogen atom and 4 or more carbon atoms.

Specific examples include 1,8-diazabicyclo[5.4.0]undecene, 1,5-diazabicyclo[4.3.0]nonene, pyridine, 2,3-lutidine, 2,4-lutidine, 2,5-lutidine, 2,6-lutidine, 3,4-lutidine, 3,5-lutidine, 2,3,4-collidine, 2,4,5-collidine, 2,5,6-collidine, 2,4,6-collidine, 3,4,5-collidine, and 3,5,6-collidine.

The inorganic base is preferably a base selected from the group consisting of a sodium salt and a potassium salt, and examples thereof include sodium carbonate, potassium carbonate, sodium bicarbonate, and potassium bicarbonate.

Further, hydrides of alkali metals are also preferred, such as sodium hydride and potassium hydride.

The base is preferably any one selected from the group consisting of sodium carbonate, potassium carbonate, sodium bicarbonate, potassium bicarbonate, lithium methoxide, lithium ethoxide, lithium tert-butoxide, sodium methoxide, sodium ethoxide, sodium tert-butoxide, potassium methoxide, potassium ethoxide, potassium tert-butoxide, triethylamine, tri-n-propylamine, tributylamine, diisopropylethylamine, N,N-diethylcyclohexylamine, 1,8-diazabicyclo[5.4.0]undecene, and 1,5-diazabicyclo[4.3.0]nonene.

Among them, sodium carbonate, potassium carbonate, sodium bicarbonate, potassium bicarbonate, sodium tert-butoxide, or potassium tert-butoxide are preferred, and potassium carbonate, potassium bicarbonate, or potassium tert-butoxide are particularly preferred. These bases can be used alone or in combination.

In another preferred aspect, when the base is an organic base, the organic base is preferably any one selected from the group consisting of trimethylamine, triethylamine, diisopropylethylamine, tri-n-propylamine, pyridine, 2,3-lutidine, 2,4-lutidine, 2,5-lutidine, 2,6-lutidine, 3,4-lutidine, 3,5-lutidine, 2,3,4-collidine, 2,4,5-collidine, 2,5,6-collidine, 2,4,6-collidine, 3,4,5-collidine, and 3,5,6-collidine.

These bases can be used alone or in combination.

An amount of the used base is not particularly limited, but is usually preferably from 0.01 mol to 20 mol, and more preferably from 0.05 mol to 5 mol, per 1 mol of a compound to be trifluoromethanesulfonylated, which will be described later.

The first composition according to the present disclosure may further contain a solvent.

The solvent is not particularly limited as long as the solvent dissolves the compound represented by Formula (1) above and the above base, and examples thereof include a reaction solvent for trifluoromethanesulfonylation to be described below.

The first composition according to the present disclosure may or may not include the solvent.

### <Method for Producing Trifluoromethanesulfonyloxy Compound>

A method for producing a trifluoromethanesulfonyloxy compound using the first composition according to the present disclosure (hereinafter, also referred to as a first production method) includes reacting the first trifluoromethanesulfonylating agent composition described above with a compound represented by the following Formula (2).

### (Compound to be Trifluoromethanesulfonylated)

The compound to be trifluoromethanesulfonylated with the first trifluoromethanesulfonylating agent composition (hereinafter also referred to as the compound to be trifluoromethanesulfonylated) is a hydroxylated aromatic compound represented by the following Formula (2).
Formula (2):

**Ar-OH** (2)

In Formula (2), Ar represents an aromatic ring group or a substituted aromatic ring group.

In a hydroxylated aromatic compound represented by Formula (2), Ar represents an aromatic ring group or a substituted aromatic ring group.

The aromatic ring group is not particularly limited, and may be a monocyclic or polycyclic ring. An aromatic ring group having 1 to 18 carbon atoms is preferred, and examples thereof include an aromatic hydrocarbon group such as a phenyl group, a naphthyl group, and an anthryl group, and an aromatic heterocyclic group containing a heteroatom such as a nitrogen atom, an oxygen atom, or a sulfur atom, such as a pyrrolyl group (including a pyrrolyl group in which nitrogen is protected), a pyridyl group, a pyrazyl group, a pyrimidyl group, a pyridazyl group, a triazyl group, a furyl group, a thienyl group, an indolyl group (including an indolyl group in which nitrogen is protected), an indazolyl group, a quinolyl group, a carbazolyl group, a pyrrolopyridyl group, a benzofuryl group, and a benzothienyl group.

In Formula (2), Ar represents an aromatic ring group, and the aromatic ring group is preferably an aromatic heterocyclic group.

The substituted aromatic ring group has any number and any combination of substituents on any carbon or nitrogen atom of the aromatic ring group. Such substituents include halogen atoms such as fluorine, chlorine, bromine, and iodine; lower alkyl groups such as a methyl group, an ethyl group, and a propyl group; lower unsaturated groups such as a vinyl group, an allyl group, and a propargyl group; lower haloalkyl groups such as a fluoromethyl group, a chloromethyl group, and a bromomethyl group; C(CF₃)₂OH (including one in which a hydroxyl group is protected); lower alkoxy groups such as a methoxy group, an ethoxy group, and a propoxy group; lower haloalkoxy groups such as a fluoromethoxy group, chloromethoxy group, and a bromomethoxy group; lower acyloxy groups such as a formyloxy group, an acetyloxy group, a propionyloxy group, and a butyryloxy group; lower alkoxycarbonyl groups such as a cyano group, a methoxycarbonyl group, an ethoxycarbonyl group, and a propoxycarbonyl group; lower alkoxycarbonyl lower alkyl groups such as a methoxycarbonylmethyl group, an ethoxycarbonyl ethyl group, and a propoxycarbonyl propyl group; aromatic ring groups such as a β-D-glucopyranoside group, a phenyl group, a naphthyl group, an anthryl group, a pyrrolyl group (including a pyrrolyl group in which nitrogen is protected), a pyridyl group, a furyl group, a thienyl group, an indolyl group (including an indolyl group in which nitrogen is protected), a quinolyl group, a benzofuryl group, and a benzothienyl group; a protected carboxyl group, an amino group, a protected amino group, lower alkylamino groups, lower alkylamino lower alkyl groups, a hydroxyl group, a protected hydroxyl group, and an X'-Ar'-OH group. These substituents may be further substituted, for example, with the above-mentioned substituents of "such substituents".

In Formula (2), Ar represents the substituted aromatic ring group, and a substituent of the substituted aromatic ring group is preferably a lower alkyl group, a lower alkoxycarbonyl lower alkyl group, a β-D-glucopyranoside group, an amino group, a lower alkylamino group, or a hydroxyl group.

X' in the X'-Ar'-OH group represents a C(CH₃)₂ group, a C(CF₃)₂ group, an oxygen atom, a nitrogen atom (including a protected nitrogen atom), a sulfur atom, an SO group, or a SO₂ group, and Ar' represents a phenylene group or a substituted phenylene group. A substitution site of the phenylene group is 2-, 3- or 4-site relative to the hydroxyl group. A substituent of the substituted phenylene group is the same as the substituent of the substituted aromatic ring group. Specific examples of the hydroxylated aromatic compound represented by Formula (2) substituted with the X'-Ar'-OH group include the following compounds.

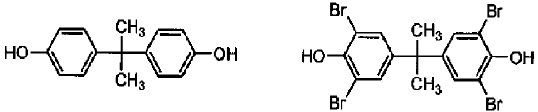

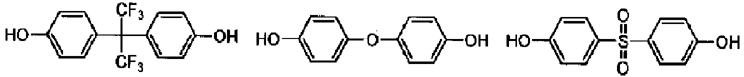

In the present specification, the term "lower" means a linear or branched chain having 1 to 6 carbon atoms, or a cyclic group (in the case of having 3 or more carbon atoms). Further, the aromatic ring group in the above "such substituents" can also be substituted with a halogen atom, a lower alkyl group, a lower unsaturated group, a lower haloalkyl group, a C(CF₃)₂OH group (including a group in which a hydroxyl group is protected), a lower alkoxy group, a lower haloalkoxy group, a formyloxy group, a lower acyloxy group, a cyano group, a lower alkoxycarbonyl group, a lower alkoxycarbonyl lower alkyl group, a protected carboxyl group, a protected amino group, a hydroxyl group, a protected hydroxyl group, an X'-Ar'-OH group, and the like. Further, protective groups for a pyrrolyl group, an indolyl group, a hydroxyl group, a carboxyl group, and an amino group are those described in Protective Groups in Organic Synthesis, Third Edition, 1999, John Wiley & Sons,Inc., and the like. Among these, an aromatic ring group and a substituted aromatic ring group excluding a "hydroxyl group", an "aromatic ring group", and an "X'-Ar'-OH group" as the substituent are preferred, and an aromatic hydrocarbon group and a substituted aromatic hydrocarbon group (aromatic hydrocarbon group having a substituent) excluding a "hydroxyl group", an "aromatic ring group", and an "X'-Ar'-OH group" as the substituent are particularly preferred. In a hydroxylated aromatic compound having a plurality of hydroxyl groups, fluorosulfonylation may occur a plurality of times depending on selected reaction conditions.

In a preferred aspect, a hydroxylated aromatic compound represented by Formula (2) has at least one selected from an alcoholic hydroxyl group and an amino group as a substituent. These substituents may be further substituted, for example, with the above-mentioned substituents of "such substituents".

Examples of the compound represented by Formula (2) are shown below, but the invention is not limited thereto.

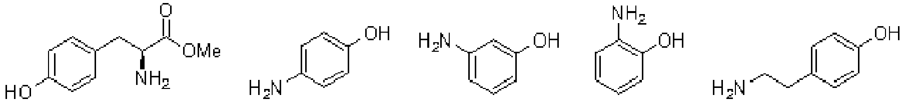

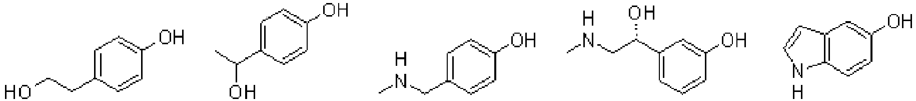

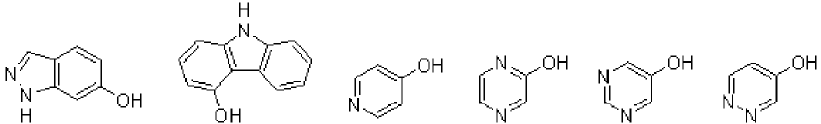

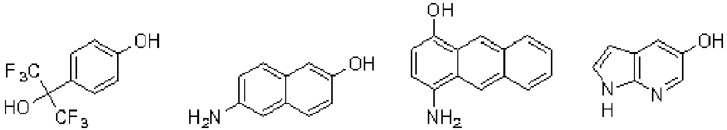

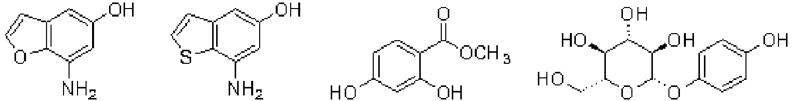

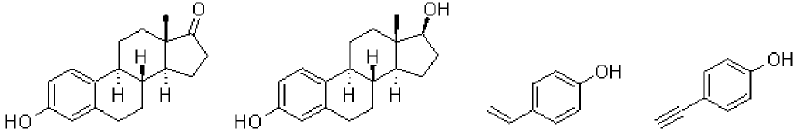

In the trifluoromethanesulfonylation reaction, the hydroxylated aromatic compound represented by Formula (2) is preferably used in an amount of 0.7 to 1.2 mol per 1.0 mol of the trifluoromethanesulfonyl compound represented by Formula (1). The amount is more preferably from 0.8 to 1.0 mol.

### (Solvent)

The above-described trifluoromethanesulfonylation reaction is preferably performed using a reaction solvent.

Examples of the reaction solvent for the trifluoromethanesulfonylation include ether solvents, aliphatic hydrocarbon solvents, aromatic hydrocarbon solvents, halogenated hydrocarbon solvents, ester solvents, amide solvents, nitrile solvents, and sulfoxide solvents.

Specific examples of these reaction solvents include ether solvents such as diethyl ether, diisopropyl ether, dibutyl ether, tert-butyl methyl ether, tetrahydrofuran, 2-methyltetrahydrofuran, tetrahydropyran, 1,4-dioxane, and cyclopentyl methyl ether.

Examples of the aliphatic hydrocarbon solvent include n-hexane, n-heptane, n-pentane, n-nonane, and n-decane.

Examples of the aromatic hydrocarbon solvents include toluene, xylene, mesitylene, and ethylbenzene.

Examples of the halogenated hydrocarbon solvents include methylene chloride, chloroform, and 1,2-dichloroethane.

Examples of the ester solvents include ethyl acetate, isopropyl acetate, n-butyl acetate, and γ-butyrolactone.

Examples of the amide solvents include N,N-dimethylformamide, N, N-dimethy acetamide, N-methylpyrrolidone, and 1,3-dimethyl-2-imidazolidinone.

Examples of the nitrile solvents include acetonitrile, propionitrile, and benzonitrile.

Examples of the sulfoxide solvents include dimethyl sulfoxide.

Among these, tetrahydrofuran, N,N-dimethylformamide, N, N-dimethy acetamide, 1,3-dimethyl-2-imidazolidinone, acetonitrile, propionitrile and dimethyl sulfoxide are preferred, and tetrahydrofuran, N,N-dimethylformamide, and acetonitrile are particularly preferred, because the compounds are easily available and have excellent solubility for the substrate and the trifluoromethanesulfonylating agent according to the present disclosure. These reaction solvents can be used alone or in combination.

An amount of the reaction solvent used in the trifluoromethanesulfonylation is not particularly limited, but it is sufficient to use 0.05 liters (L) or more per 1 mol of the compound to be trifluoromethanesulfonylated, usually from 0.1 to 20 L is preferred, and particularly from 0.1 to 10 L is more preferred.

### (Reaction Temperature)

A reaction temperature for the trifluoromethanesulfonylation is not particularly limited, and the trifluoromethanesulfonylation is preferably carried out at a reaction temperature of 150°C or less, more preferably in a range of from -100 to 150°C, and still more preferably in a range of from -78 to 100°C.

### (Reaction Time)

A reaction time for trifluoromethanesulfonylation is not particularly limited, but may be within a range of from 0.1 to 72 hours. Since the reaction time varies depending on raw materials and reaction conditions, it is preferable to follow progress of the reaction by an analytical method such as gas chromatography, liquid chromatography, or NMR and to end the reaction when the raw materials have almost disappeared.

### (Post-treatment Operation: Liquid Separation)

After the above reaction, as a post-treatment operation for isolating the trifluoromethanesulfonyloxy compound, a general operation in organic synthesis may be carried out.

For example, it is preferable to post-treat a reaction solution containing the trifluoromethanesulfonyloxy compound after completion of the reaction with water, an acidic aqueous solution, or an alkali aqueous solution. That is, the reaction solution after the completion of the reaction may be diluted with an organic solvent, washed with water, an aqueous solution of a mineral acid (inorganic acid), or an aqueous solution of alkali metal salts, and a reaction mixture (organic phase) may be concentrated.

Examples of the organic solvent for the post-treatment include ether solvents, aliphatic hydrocarbon solvents, aromatic hydrocarbon solvents, halogenated hydrocarbon solvents, and ester solvents.

Specific examples of the organic solvents for the post-treatment include diethyl ether, diisopropyl ether, dibutyl ether, tert-butyl methyl ether, tetrahydrofuran, 2-methyltetrahydrofuran, tetrahydropyran, cyclopentyl methyl ether, n-hexane, n-heptane, n-pentane, n-nonane, n-decane, toluene, xylene, mesitylene, ethylbenzene, methylene chloride, chloroform, 1,2-dichloroethane, ethyl acetate, and n-butyl acetate.

Among these, diisopropyl ether, dibutyl ether, tert-butyl methyl ether, 2-methyltetrahydrofuran, cyclopentyl methyl ether, toluene, xylene, mesitylene, ethylbenzene, methylene chloride, chloroform, 1,2-dichloroethane, ethyl acetate, and n-butyl acetate are preferred, and ethyl acetate is particularly preferred. These reaction solvents can be used alone or in combination.

An amount of the solvent for the post-treatment is not particularly limited, but it is sufficient to use 0.05 liters (L) or more per 1 mol of the compound to be trifluoromethanesulfonylated, usually from 0.1 to 20 L is preferred, and particularly from 0.1 to 10 L is more preferred.

Specific examples of the mineral acids for the post-treatment include hydrochloric acid, hydrobromic acid, sulfuric acid, phosphoric acid, and nitric acid. Among these, hydrochloric acid and sulfuric acid are preferred, and hydrochloric acid is particularly preferred.

Specific examples of the alkali metal salts for the post-treatment include sodium bicarbonate, potassium bicarbonate, sodium carbonate, sodium hydroxide, potassium hydroxide, or potassium carbonate.

The obtained trifluoromethanesulfonyloxy compound can be suitably and preferably used, for example, in a coupling reaction using transition metals.

As described above, since the trifluoromethanesulfonyloxy compound obtained by the first production method according to the present disclosure can be isolated from the reaction solution after the completion of the reaction by simply carrying out the simple post-treatment operation, the method can be carried out on an industrial scale, and as a result, it becomes possible to produce a coupling reaction product much more efficiently than by a method in the related art.

### · Second Aspect

Another preferred aspect of the present disclosure is a trifluoromethanesulfonylating agent composition containing the compound represented by the above Formula (11).

By using the trifluoromethanesulfonylating agent composition containing the compound represented by Formula (11) (hereinafter also referred to as a second trifluoromethanesulfonylating agent composition, or simply as a second composition), not only a substrate having a phenolic hydroxyl group but also a wide range of substrates can be trifluoromethanesulfonylated, and trifluoromethanesulfonyl compounds derived from these substrates can be easily provided under industrially feasible conditions.

The second composition according to the present disclosure includes a triflylimidazolium salt or a triflyltriazolium salt, which is a compound represented by Formula (11). Two or more of these may be used in combination.

The second composition preferably contains the compound represented by Formula (11) in an amount of 80% or more, and more preferably 90% or more, based on a total mass of the second composition.

The second composition according to the present disclosure may further contain a base.

Examples of the base include a base which can be used in the trifluoromethanesulfonylation to be described below.

The second composition according to the present disclosure may or may not include the base.

An amount of the used base is not particularly limited, but is usually preferably from 0.01 to 20 mol, and more preferably from 0.05 to 5 mol, per 1 mol of a compound to be trifluoromethanesulfonylated, which will be described later.

The second composition according to the present disclosure may further contain a solvent.

The solvent is not particularly limited as long as the solvent dissolves the compound represented by Formula (11) and, in the case in which the second composition contains a base, the base, and examples thereof include a reaction solvent for the trifluoromethanesulfonylation to be described below.

The second composition according to the present disclosure may or may not include the solvent.

### <Method for Producing Trifluoromethanesulfonyl Compound>

A method for producing a trifluoromethanesulfonyl compound using the second composition according to the present disclosure (hereinafter, also referred to as a second production method) includes reacting the second trifluoromethanesulfonylating agent composition described above with the compound to be trifluoromethanesulfonylated.

### (Compound to be Trifluoromethanesulfonylated)

The compound to be trifluoromethanesulfonylated with the second trifluoromethanesulfonylating agent composition (hereinafter also referred to as the compound to be trifluoromethanesulfonylated) is preferably at least one substrate selected from the group consisting of a compound having a phenolic hydroxyl group, a compound having an alcoholic hydroxyl group, a ketone, a primary amine, and a secondary amine.

An example of the compound having a phenolic hydroxyl group is a hydroxylated aromatic compound represented by the above Formula (2).

Examples of the compound having an alcoholic hydroxyl group include an alkyl alcohol having 1 to 20 carbon atoms, methyl lactate, phenylethyl alcohol, and tetraacetyl-β-D-mannose.

Examples of the ketone include ethyl acetoacetate, acetylacetone, cyclohexanone, 1,3-cyclohexanedione, and ethyl 2-oxocyclohexanecarboxylate.

Examples of the primary amine include alkylamines having 1 to 20 carbon atoms, aniline, 1-phenylethylamine, α-amino acids, and the like.

Examples of the secondary amine include alkylamines having 1 to 20 carbon atoms, methylaniline, N-methylphenylethylamine, piperidine, and the like.

For the above-mentioned compound to be trifluoromethanesulfonylated, not only those having one functional group in one molecule but also those having two or three functional groups in one molecule, can be trifluoromethanesulfonylated by the second trifluoromethanesulfonylating agent composition according to the present disclosure. In a case of a compound to be trifluoromethanesulfonylated having a plurality of functional groups, fluorosulfonylation may occur a plurality of times depending on selected reaction conditions.

In the trifluoromethanesulfonylation reaction in which the compound to be trifluoromethanesulfonylated are allowed to come into contact with the second trifluoromethanesulfonylating agent composition according to the present disclosure to perform the trifluoromethanesulfonylation, a ratio of the compound to be trifluoromethanesulfonylated to the trifluoromethanesulfonylating agent (compound represented by Formula (11)) is preferably the compound to be trifluoromethanesulfonylated:trifluoromethanesulfonylating agent = 1:0.5 to 1:5.0, and more preferably 1:1 to 1:3.0, expressed as a molar ratio.

### (Solvent)

The above-described trifluoromethanesulfonylation reaction is preferably performed using a reaction solvent.

Examples of the reaction solvent for the trifluoromethanesulfonylation include ether solvents, aliphatic hydrocarbon solvents, aromatic hydrocarbon solvents, halogenated hydrocarbon solvents, ester solvents, amide solvents, nitrile solvents, and sulfoxide solvents.

Specific examples of these reaction solvents include those described above as the reaction solvent in the first production method, and preferred examples are also the same.

An amount of the reaction solvent used in the trifluoromethanesulfonylation is not particularly limited, but it is sufficient to use 0.05 liters (L) or more per 1 mol of the compound to be trifluoromethanesulfonylated, usually from 0.1 to 20 L is preferred, and particularly from 0.1 to 10 L is more preferred.

### (Base)

The above-described trifluoromethanesulfonylation reaction is preferably performed using a base.

Examples of the base include the bases described above as the base which can be contained in the first composition.

The base is preferably triethylamine, sodium hydride, diisopropylethylamine, tributylamine, pyridine, 2,3-lutidine, 2,4-lutidine, 2,5-lutidine, 2,6-lutidine, 3,4-lutidine, or 3,5-lutidine, and more preferably triethylamine or sodium hydride.

An amount of the used base is not particularly limited, but is usually preferably from 0.01 mol to 20 mol, and more preferably from 0.05 mol to 5 mol, per 1 mol of the compound to be trifluoromethanesulfonylated, which will be described later.

### (Reaction Temperature)

A reaction temperature for the trifluoromethanesulfonylation is not particularly limited, and the trifluoromethanesulfonylation is preferably carried out at a reaction temperature of 150°C or less, more preferably in a range of from -100 to 150°C, and still more preferably in a range of from -78 to 100°C.

### (Reaction Time)

A reaction time for the trifluoromethanesulfonylation is not particularly limited, but may be within a range of from 0.1 to 72 hours. Since the reaction time varies depending on raw materials and reaction conditions, it is preferable to follow progress of the reaction by an analytical method such as gas chromatography, liquid chromatography, or NMR and to end the reaction when the raw materials have almost disappeared.

### (Post-treatment Operation: Liquid Separation)

After the above reaction, as a post-treatment operation for isolating the trifluoromethanesulfonyl compound, a general operation in organic synthesis may be carried out.

For example, it is preferable to post-treat a reaction solution containing the trifluoromethanesulfonyl compound after completion of the reaction with water, an acidic aqueous solution, or an alkali aqueous solution. That is, the reaction solution after the completion of the reaction may be diluted with an organic solvent, washed with water, an aqueous solution of a mineral acid (inorganic acid), or an aqueous solution of alkali metal salts, and a reaction mixture (organic phase) may be concentrated.

Examples of the organic solvent for the post-treatment include ether solvents, aliphatic hydrocarbon solvents, aromatic hydrocarbon solvents, halogenated hydrocarbon solvents, and ester solvents.

Specific examples of the organic solvent for the post-treatment include those described above as the organic solvent for the post-treatment in the first production method, and preferred examples are also the same. These organic solvents can be used alone or in combination.

An amount of the solvent for the post-treatment is not particularly limited, but it is sufficient to use 0.05 liters (L) or more per 1 mol of the compound to be trifluoromethanesulfonylated, usually from 0.1 to 20 L is preferred, and particularly from 0.1 to 10 L is more preferred.

Specific examples of the mineral acid and the alkali metal salts for the post-treatment include those described above for the mineral acid and alkali metal salt for the post-treatment in the first production method, and preferred examples are also the same.

As described above, the trifluoromethanesulfonyl compound obtained by the second production method according to the present disclosure can be isolated from the reaction solution after the completion of the reaction by simply carrying out the simple post-treatment operation, and therefore can be produced industrially and efficiently.

### Examples

Hereinafter, the present disclosure will be described in detail with reference to Examples, but the present disclosure is not limited to these Examples. Here, in Examples 1 to 22 and 28 to 35, a yield (%) is a value obtained by measuring a nuclear magnetic resonance spectrum ¹⁹F-NMR.

In the following, Synthesis Examples 1 to 3 and Examples 1 to 27 correspond to the first aspect using the trifluoromethanesulfonylating agent composition containing the compound represented by the above Formula (1) and the specific base. Further, Synthesis Examples 4 to 7 and Examples 28 to 35 correspond to the second aspect using the trifluoromethanesulfonylating agent composition containing the compound represented by the above Formula (11).

### (Synthesis Example 1)

After collecting 5.0 g (73 mmol, 1.0 eq. = molar equivalent ratio, the same applies below) of imidazole, 23.5 g (139 mmol, 1.9 eq.) of trifluoromethanesulfonyl chloride, 15.6 g (122 mmol, 2.0 eq.) of sodium carbonate, and 73 mL of acetonitrile as a reaction solvent, a mixture was charged into a 200 mL-sized three-neck flask equipped with a stirrer. Thereafter, the mixture was stirred at a room temperature (about 25°C, the same applies below) for 16 hours. A small amount of a reaction solution was concentrated and analyzed by ¹H-NMR. A signal derived from imidazole completely disappeared, and a new signal derived from 1-trifluoromethanesulfonylimidazole was observed.

The reaction solution was filtered through celite (Celite, registered trademark: diatomaceous earth calcined with sodium carbonate, the same applies hereinafter) to remove insoluble matter, and acetonitrile was removed from a filtrate to obtain 9.7 g of 1-trifluoromethanesulfonylimidazole.

### [Physical Properties]

1-trifluoromethanesulfonylimidazole;
¹H-NMR(400MHz,CD₃CN)δ(ppm):8.15(1H,s),7.56(1H,s),7.29(1H,s)
¹⁹F-NMR(373MHz,CD₃CN)δ(ppm):-77.3(3F,s)

Hereinafter, a reaction in Synthesis Example 1 will be shown.

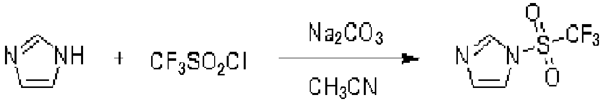

### (Synthesis Example 2)

After collecting 5.0 g (61 mmol, 1.0 eq.) of 2-methylimidazole, 19.5 g (116 mmol, 1.9 eq.) of trifluoromethanesulfonyl chloride, 12.9 g (122 mmol, 2.0 eq.) of sodium carbonate, and 61 mL of acetonitrile as a reaction solvent were collected, a mixture was charged into a 200 mL-sized three-neck flask equipped with a stirrer. Thereafter, the mixture was stirred at a room temperature for 22 hours. A small amount of a reaction solution was concentrated and analyzed by ¹H-NMR. A signal derived from 2-methylimidazole completely disappeared, and a new signal derived from 1-trifluoromethanesulfonyl-2-methylimidazole was observed.

The reaction solution was filtered through celite to remove insoluble matter, and acetonitrile was removed from a filtrate to obtain 10.2 g of 1-trifluoromethanesulfonyl-2-methylimidazole.

### [Physical Properties]

1-trifluoromethanesulfonyl-2-methylimidazole;
¹H-NMR(400MHz,CD₃CN)δ(ppm):7.42(1H,s),7.05(1H,s),2.56(3H,s)
¹⁹F-NMR(373MHz,CD₃CN)δ(ppm):-76.7(3F,s)

Hereinafter, a reaction in Synthesis Example 2 will be shown.

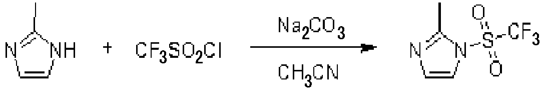

### (Synthesis Example 3)

After collecting 5.0 g (44 mmol) of 4-nitroimidazole and 45 mL of dichloromethane as a reaction solvent, a mixture was charged into a 100 mL-sized three-neck flask equipped with a stirring bar, which served as a reactor. Subsequently, 6.3 g (22 mmol, 0.5 eq.) of trifluoromethanesulfonic anhydride was added dropwise, and a mixture was stirred at a room temperature for 21 hours. A reaction solution was filtered through celite to remove insoluble matter, and dichloromethane was removed from a filtrate, thereby obtaining 5.2 g of 1-trifluoromethanesulfonyl-4-nitroimidazole.

### [Physical Properties]

1-trifluoromethanesulfonyl-4-nitroimidazole;
¹H-NMR(400MHz,CDCl₃)δ(ppm):8.17(1H,d,J=1.2Hz),8.02(1H,d,J=1.6Hz)
¹⁹F-NMR(373MHz,CDCl₃)δ(ppm):-74.6(3F,s)

Hereinafter, a reaction in Synthesis Example 3 will be shown.

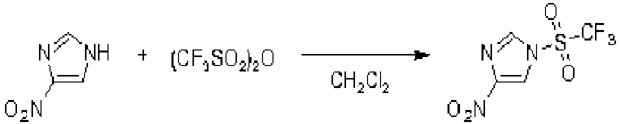

### [Example 1]

After collecting 195 mg (1.00 mmol, 1.0 eq.) of L-tyrosine methyl ester as a substrate and 5 mL of acetonitrile as a reaction solvent, a mixture was charged into a 20 mL-sized Schlenk tube equipped with a stirring bar as a reactor. Subsequently, a composition containing both components, that is, 220 mg (1.10 mmol, 1.1 eq.) of 1-trifluoromethanesulfonylimidazole and 28 mg (0.20 mmol, 0.2 eq.) of potassium carbonate, was added to the reactor, and a mixture was stirred at a room temperature for 2 hours to obtain a reaction solution. Benzotrifluoride was added to the reaction solution as an internal standard, and when an analysis was performed by ¹⁹F-NMR, a signal derived from methyl 2-amino-3-(4-trifluoromethanesulfonyloxyphenyl)propanoate was found at -74 ppm, and a quantitative yield thereof was 100%.

Hereinafter, a reaction in Example 1 will be shown.

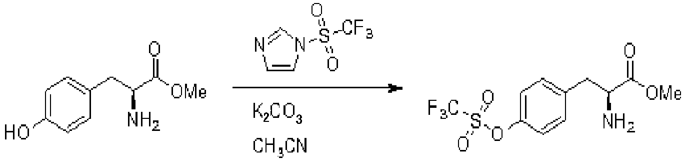

### [Example 2]

After collecting 196 mg (1.00 mmol, 1.0 eq.) of L-tyrosine methyl ester as a substrate and 5 mL of dimethylformamide (DMF) as a reaction solvent, a mixture was charged into a 20 mL-sized Schlenk tube equipped with a stirring bar as a reactor. Subsequently, a composition containing both components, that is, 228 mg (1.14 mmol, 1.1 eq.) of 1-trifluoromethanesulfonylimidazole and 27 mg (0.20 mmol, 0.2 eq.) of potassium carbonate, was added to the reactor, and a mixture was stirred at room temperature for 2 hours to obtain a reaction solution. Benzotrifluoride was added to the reaction solution as an internal standard, and when an analysis was performed by ¹⁹F-NMR, a signal derived from methyl 2-amino-3-(4-trifluoromethanesulfonyloxyphenyl)propanoate was found at -74 ppm, and a quantitative yield thereof was 100%.

Hereinafter, a reaction in Example 2 will be shown.

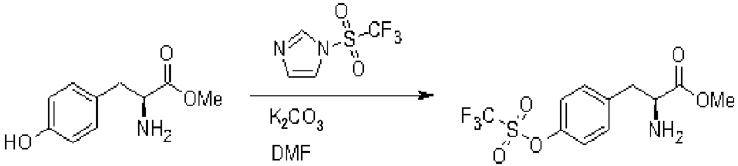

### [Example 3]

After collecting 197 mg (1.01 mmol, 1.0 eq.) of L-tyrosine methyl ester as a substrate and 5 mL of dimethyl sulfoxide (DMSO) as a reaction solvent, a mixture was charged into a 20 mL-sized Schlenk tube equipped with a stirring bar as a reactor. Subsequently, a composition containing both components, that is, 228 mg (1.14 mmol, 1.1 eq.) of 1-trifluoromethanesulfonylimidazole and 28 mg (0.20 mmol, 0.2 eq.) of potassium carbonate, was added to the reactor, and a mixture was stirred at a room temperature for 2 hours to obtain a reaction solution. Benzotrifluoride was added to the reaction solution as an internal standard, and when an analysis was performed by ¹⁹F-NMR, a signal derived from methyl 2-amino-3-(4-trifluoromethanesulfonyloxyphenyl)propanoate was found at -74 ppm, and a quantitative yield thereof was 100%.

Hereinafter, a reaction in Example 3 will be shown.

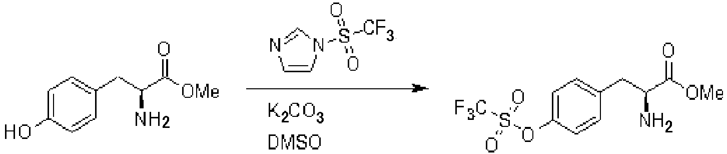

### [Example 4]

After collecting 196 mg (1.00 mmol, 1.0 eq.) of L-tyrosine methyl ester as a substrate and 5 mL of gamma-butyrolactone as a reaction solvent, a mixture was charged into a 20 mL-sized Schlenk tube equipped with a stirring bar as a reactor. Subsequently, a composition containing both components, that is, 223 mg (1.12 mmol, 1.1 eq.) of 1-trifluoromethanesulfonylimidazole and 27 mg (0.20 mmol, 0.2 eq.) of potassium carbonate, was added to the reactor, and a mixture was stirred at a room temperature for 2 hours to obtain a reaction solution. Benzotrifluoride was added to the reaction solution as an internal standard, and when an analysis was performed by ¹⁹F-NMR, a signal derived from methyl 2-amino-3-(4-trifluoromethanesulfonyloxyphenyl)propanoate was found at -74 ppm, and a quantitative yield thereof was 100%.

Hereinafter, a reaction in Example 4 will be shown.

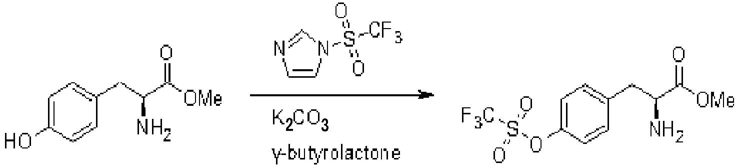

### [Example 5]

After collecting 586 mg (3.00 mmol, 1.0 eq.) of L-tyrosine methyl ester as a substrate and 15 mL of tetrahydrofuran (THF) as a reaction solvent, a mixture was charged into a 50 mL-sized two-neck flask equipped with a stirring bar as a reactor. Subsequently, a composition containing both components, that is, 720 mg (3.60 mmol, 1.2 eq.) of 1-trifluoromethanesulfonylimidazole and 67 mg (0.60 mmol, 0.2 eq.) of potassium tert-butoxide, was added to the reactor, and a mixture was stirred at a room temperature for 16 hours to obtain a reaction solution. Benzotrifluoride was added to the reaction solution as an internal standard, and when an analysis was performed by ¹⁹F-NMR, a signal derived from methyl 2-amino-3-(4-trifluoromethanesulfonyloxyphenyl)propanoate was found at -74 ppm, and a quantitative yield thereof was 99%.

Hereinafter, a reaction in Example 5 will be shown.

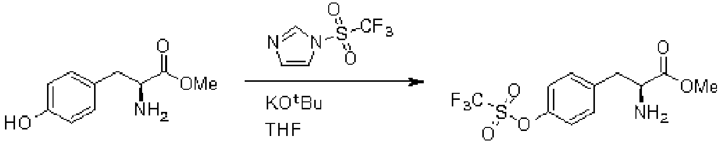

### [Example 6]

After collecting 196 mg (1.01 mmol, 1.0 eq.) of L-tyrosine methyl ester as a substrate and 5 mL of acetonitrile as a reaction solvent, a mixture was charged into a 20 mL-sized Schlenk tube equipped with a stirring bar as a reactor. Subsequently, a composition containing both components, that is, 222 mg (1.11 mmol, 1.1 eq.) of 1-trifluoromethanesulfonylimidazole and 51 mg (0.52 mmol, 0.5 eq.) of triethylamine, was added to the reactor, and a mixture was stirred at a room temperature for 20 hours to obtain a reaction solution. Benzotrifluoride was added to the reaction solution as an internal standard, and when an analysis was performed by ¹⁹F-NMR, a signal derived from methyl 2-amino-3-(4-trifluoromethanesulfonyloxyphenyl)propanoate was found at -74 ppm, a quantitative yield thereof was 42%, and no side reaction was observed. Specifically, no signal derived from "methyl 2-trifluoromethanesulfonylamino-3-(4-trifluoromethanesulfonyloxyphenyl)propanoate" which may correspond to a by-product could be found.

Hereinafter, a reaction in Example 6 will be shown.

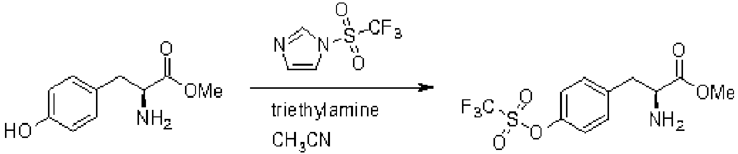

### [Example 7]

After collecting 111 mg (1.02 mmol, 1.0 eq.) of 4-aminophenol as a substrate and 5 mL of acetonitrile as a reaction solvent, a mixture was charged into a 20 mL-sized Schlenk tube equipped with a stirring bar as a reactor. Subsequently, a composition containing both components, that is, 222 mg (1.11 mmol, 1.1 eq.) of 1-trifluoromethanesulfonylimidazole and 28 mg (0.20 mmol, 0.2 eq.) of potassium carbonate, was added to the reactor, and a mixture was stirred at a room temperature for 2 hours to obtain a reaction solution. Benzotrifluoride was added to the reaction solution as an internal standard, and when an analysis was performed by ¹⁹F-NMR, a signal derived from 4-aminophenyl trifluoromethanesulfonate was found at -74 ppm, and a quantitative yield thereof was 100%.

Hereinafter, a reaction in Example 7 will be shown.

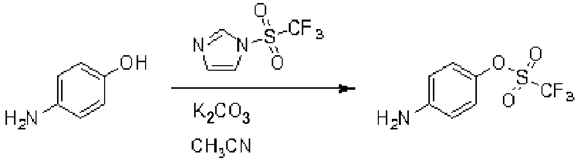

### [Example 8]

After collecting 109 mg (1.00 mmol, 1.0 eq.) of 2-aminophenol as a substrate and 5 mL of acetonitrile as a reaction solvent, a mixture was charged into a 20 mL-sized Schlenk tube equipped with a stirring bar as a reactor. Subsequently, a composition containing both components, that is, 202 mg (1.01 mmol, 1.0 eq.) of 1-trifluoromethanesulfonylimidazole and 29 mg (0.21 mmol, 0.2 eq.) of potassium carbonate, was added to the reactor, and a mixture was stirred at room temperature for 3 hours to obtain a reaction solution. Benzotrifluoride was added to the reaction solution as an internal standard, and when an analysis was performed by ¹⁹F-NMR, a signal derived from 2-aminophenyl trifluoromethanesulfonate was found at -75 ppm, and a quantitative yield thereof was 48%. A signal derived from 1,1,1-trifluoro-N-(2-hydroxyphenyl)methanesulfonamide was found at -78 ppm, and a quantitative yield thereof was 17%.

Hereinafter, a reaction in Example 8 will be shown.

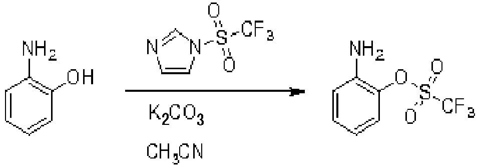

### [Example 9]

After collecting 1.08 g (7.87 mmol, 1.0 eq.) of 4-(2-aminoethyl)phenol as a substrate and 39 mL of tetrahydrofuran as a reaction solvent, a mixture was charged into a 20 mL-sized Schlenk tube equipped with a stirring bar as a reactor. Subsequently, a composition containing both components, that is, 1.89 g (9.44 mmol, 1.2 eq.) of 1-trifluoromethanesulfonylimidazole and 177 mg (1.58 mmol, 0.2 eq.) of potassium tert-butoxide, was added to the reactor, and a mixture was stirred at room temperature for 3 hours to obtain a reaction solution. Benzotrifluoride was added to the reaction solution as an internal standard, and when an analysis was performed by ¹⁹F-NMR, a signal derived from 4-(2-aminoethyl)phenyl trifluoromethanesulfonate was found at -75 ppm, and a quantitative yield thereof was 97%.

Hereinafter, a reaction in Example 9 will be shown.

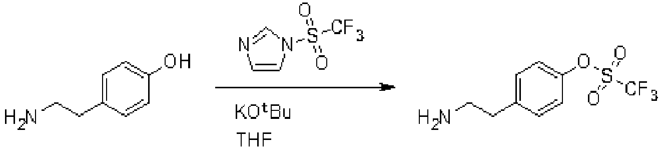

### [Example 10]

After collecting 276 mg (2.00 mmol, 1.0 eq.) of 2-(4-hydroxyphenyl)ethanol as a substrate and 10 mL of acetonitrile as a reaction solvent, a mixture was charged into a 20 mL-sized Schlenk tube equipped with a stirring bar as a reactor. Subsequently, a composition containing both components, that is, 401 mg (2.00 mmol, 1.0 eq.) of 1-trifluoromethanesulfonylimidazole and 14 mg (0.10 mmol, 0.05 eq.) of potassium carbonate, was added to the reactor, and a mixture was stirred at room temperature for 22 hours to obtain a reaction solution. Benzotrifluoride was added to the reaction solution as an internal standard, and when an analysis was performed by ¹⁹F-NMR, a signal derived from 4-(2-hydroxyethyl)phenyl trifluoromethanesulfonate was found at -74 ppm, and a quantitative yield thereof was 92%.

Hereinafter, a reaction in Example 10 will be shown.

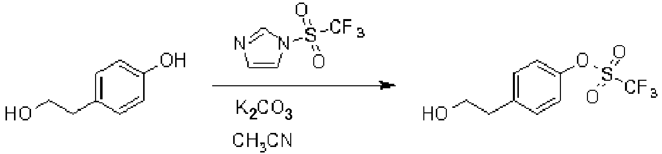

### [Example 11]

After collecting 139 mg (1.00 mmol, 1.0 eq.) of 2-(4-hydroxyphenyl)ethanol as a substrate and 5 mL of acetonitrile as a reaction solvent, a mixture was charged into a 20 mL-sized Schlenk tube equipped with a stirring bar as a reactor. Subsequently, a composition containing both components, that is, 202 mg (1.01 mmol, 1.0 eq.) of 1-trifluoromethanesulfonylimidazole and 20 mg (0.20 mmol, 0.2 eq.) of potassium bicarbonate, was added to the reactor, and a mixture was stirred at room temperature for 30 hours to obtain a reaction solution. Benzotrifluoride was added to the reaction solution as an internal standard, and when an analysis was performed by ¹⁹F-NMR, a signal derived from 4-(2-hydroxyethyl)phenyl trifluoromethanesulfonate was found at -74 ppm, and a quantitative yield thereof was 88%.

Hereinafter, a reaction in Example 11 will be shown.

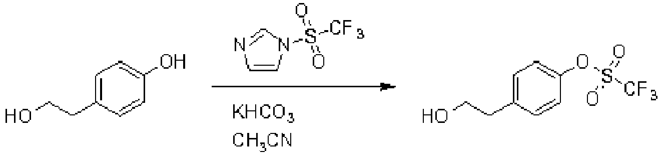

### [Example 12]

After collecting 335 mg (2.00 mmol, 1.0 eq.) of L-phenylephrine as a substrate and 10 mL of dimethylformamide as a reaction solvent, a mixture was charged into a 20 mL-sized Schlenk tube equipped with a stirring bar as a reactor. Subsequently, a composition containing both components, that is, 400 mg (2.00 mmol, 1.0 eq.) of 1-trifluoromethanesulfonylimidazole and 14 mg (0.10 mmol, 0.05 eq.) of potassium carbonate, was added to the reactor, and a mixture was stirred at room temperature for 5 hours to obtain a reaction solution. Benzotrifluoride was added to the reaction solution as an internal standard, and when an analysis was performed by ¹⁹F-NMR, a signal derived from 3-[(1R)-1-hydroxy-2-(methylamino)ethyl]phenyl trifluoromethanesulfonate was found at -74 ppm, and a quantitative yield thereof was 90%.

Hereinafter, a reaction in Example 12 will be shown.

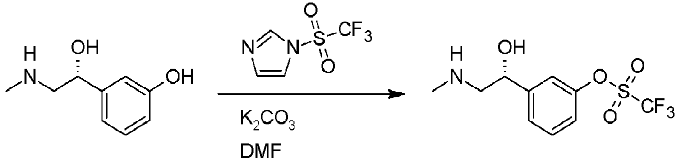

### [Example 13]

After collecting 167 mg (1.00 mmol, 1.0 eq.) of L-phenylephrine as a substrate and 5 mL of dimethylformamide as a reaction solvent, a mixture was charged into a 20 mL-sized Schlenk tube equipped with a stirring bar as a reactor. Subsequently, a composition containing both components, that is, 201 mg (1.00 mmol, 1.0 eq.) of 1-trifluoromethanesulfonylimidazole and 20 mg (0.20 mmol, 0.2 eq.) of potassium bicarbonate, was added to the reactor, and a mixture was stirred at room temperature for 22 hours to obtain a reaction solution. Benzotrifluoride was added to the reaction solution as an internal standard, and when an analysis was performed by ¹⁹F-NMR, a signal derived from 3-[(1R)-1-hydroxy-2-(methylamino)ethyl]phenyl trifluoromethanesulfonate was found at -74 ppm, and a quantitative yield thereof was 89%.

Hereinafter, a reaction in Example 13 will be shown.

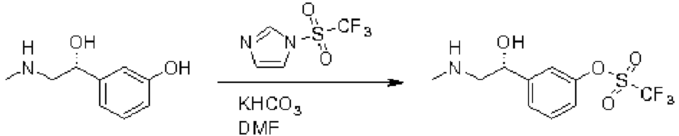

### [Example 14]

After collecting 666 mg (5.00 mmol, 1.0 eq.) of 5-hydroxyindole as a substrate and 10 mL of acetonitrile as a reaction solvent, a mixture was charged into a 20 mL-sized Schlenk tube equipped with a stirring bar as a reactor. Subsequently, a composition containing both components, that is, 1.00 g (5.00 mmol, 1.0 eq.) of 1-trifluoromethanesulfonylimidazole and 138 mg (1.00 mmol, 0.2 eq.) of potassium carbonate, was added to the reactor, and a mixture was stirred at room temperature for 3 hours to obtain a reaction solution. Benzotrifluoride was added to the reaction solution as an internal standard, and when an analysis was performed by ¹⁹F-NMR, a signal derived from indol-5-yl trifluoromethanesulfonate was found at -74 ppm, and a quantitative yield thereof was 87%. Signals derived from 1-(trifluoromethanesulfonyl)indol-5-yl trifluoromethanesulfonate were found at -74 ppm and -76 ppm, and a quantitative yield thereof was 5%.

Hereinafter, a reaction in Example 14 will be shown.

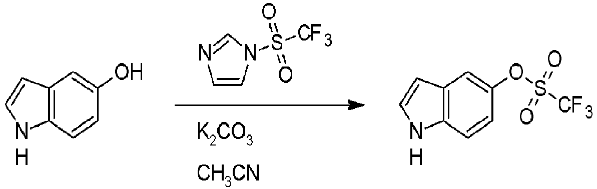

### [Example 15]

After collecting 134 mg (1.00 mmol, 1.0 eq.) of 6-hydroxyindazole as a substrate and 5 mL of acetonitrile as a reaction solvent, a mixture was charged into a 20 mL-sized Schlenk tube equipped with a stirring bar as a reactor. Subsequently, a composition containing both components, that is, 201 mg (1.01 mmol, 1.0 eq.) of 1-trifluoromethanesulfonylimidazole and 27 mg (0.19 mmol, 0.2 eq.) of potassium carbonate, was added to the reactor, and a mixture was stirred at a room temperature for 2 hours to obtain a reaction solution. Benzotrifluoride was added to the reaction solution as an internal standard, and when an analysis was performed by ¹⁹F-NMR, a signal derived from indazole-6-yl trifluoromethanesulfonate was found at -74 ppm, and a quantitative yield thereof was 74%. Signals derived from 1-(trifluoromethanesulfonyl)indazole-6-yl trifluoromethanesulfonate were found at -73 ppm and -75 ppm, and a quantitative yield thereof was 6%. A signal derived from 1-(trifluoromethanesulfonyl)indazol-6-ol was found at -75 ppm, and a quantitative yield thereof was 7%.

Hereinafter, a reaction in Example 15 will be shown.

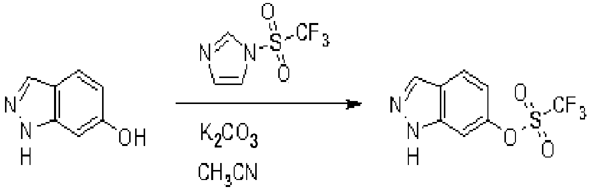

### [Example 16]

After collecting 500 mg (2.73 mmol, 1.0 eq.) of 4-hydroxycarbazole as a substrate and 5.4 mL of acetonitrile as a reaction solvent, a mixture was charged into a 20 mL-sized Schlenk tube equipped with a stirring bar as a reactor. Subsequently, a composition containing both components, that is, 550 mg (2.75 mmol, 1.0 eq.) of 1-trifluoromethanesulfonylimidazole and 75 mg (0.54 mmol, 0.2 eq.) of potassium carbonate, was added to the reactor, and a mixture was stirred at room temperature for 4 hours to obtain a reaction solution. Benzotrifluoride was added to the reaction solution as an internal standard, and when an analysis was performed by ¹⁹F-NMR, a signal derived from carbazole-4-yl trifluoromethanesulfonate was found at -72 ppm, and a quantitative yield thereof was 98%.

Hereinafter, a reaction in Example 16 will be shown.

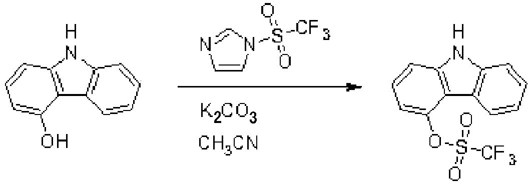

### [Example 17]

After collecting 96 mg (1.0 mmol, 1.0 eq.) of 4-hydroxypyridine as a substrate and 5 mL of tetrahydrofuran as a reaction solvent, a mixture was charged into a 20 mL-sized Schlenk tube equipped with a stirring bar as a reactor. Subsequently, a composition containing both components, that is, 244 mg (1.22 mmol, 1.2 eq.) of 1-trifluoromethanesulfonylimidazole and 22 mg (0.20 mmol, 0.2 eq.) of potassium tert-butoxide, was added to the reactor, and a mixture was stirred at 70°C for 5 hours to obtain a reaction solution. Benzotrifluoride was added to the reaction solution as an internal standard, and when an analysis was performed by ¹⁹F-NMR, a signal derived from pyridine-4-yl trifluoromethanesulfonate was found at -74 ppm, and a quantitative yield thereof was 67%, but no side reactions were observed. Specifically, no signal derived from 1-(trifluoromethanesulfonyl)-4-[(trifluoromethanesulfonyl)oxy]pyridinium which may correspond to a by-product could be found.

Hereinafter, a reaction in Example 17 will be shown.

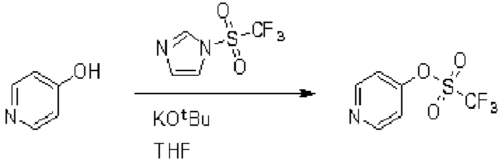

### [Example 18]

After collecting 671 mg (5.00 mmol, 1.0 eq.) of pyrrolo[2,3-b]pyridine-5-ol as a substrate and 25 mL of tetrahydrofuran as a reaction solvent, a mixture was charged into a 50 mL-sized two-neck flask equipped with a stirring bar as a reactor. Subsequently, a composition containing both components, that is, 1.20 g (6.00 mmol, 1.2 eq.) of 1-trifluoromethanesulfonylimidazole and 112 mg (1.00 mmol, 0.2 eq.) of potassium tert-butoxide, was added to the reactor, and a mixture was stirred at room temperature for 16 hours to obtain a reaction solution. Benzotrifluoride was added to the reaction solution as an internal standard, and when an analysis was performed by ¹⁹F-NMR, a signal derived from pyrrolo[2,3-b]pyridine-5-yl trifluoromethanesulfonate was found at -74 ppm, and a quantitative yield thereof was 91%, but no side reaction was observed. Specifically, no signal derived from 1-(trifluoromethanesulfonyl)-pyrrolo[2,3-b]pyridine-5-yl trifluoromethanesulfonate which may correspond to a by-product could be observed.

Hereinafter, a reaction in Example 18 will be shown.

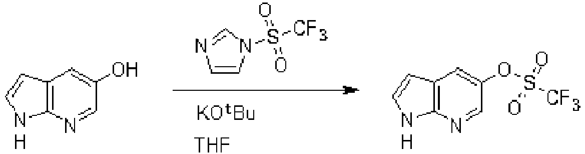

### [Example 19]

After collecting 169 mg (1.01 mmol, 1.0 eq.) of methyl 2,4-dihydroxybenzoate as a substrate and 5 mL of acetonitrile as a reaction solvent, a mixture was charged into a 20 mL-sized Schlenk tube equipped with a stirring bar as a reactor. Subsequently, a composition containing both components, that is, 202 mg (1.01 mmol, 1.0 eq.) of 1-trifluoromethanesulfonylimidazole and 28 mg (0.20 mmol, 0.2 eq.) of potassium carbonate, was added to the reactor, and a mixture was stirred at room temperature for 7 hours to obtain a reaction solution. Benzotrifluoride was added to the reaction solution as an internal standard, and when an analysis was performed by ¹⁹F-NMR, a signal derived from methyl 2-hydroxy-4-(trifluoromethanesulfonyloxy)benzoate was found at -74 ppm, and a quantitative yield thereof was 76%. A signal derived from methyl 4-hydroxy-2-[(trifluoromethanesulfonyl)oxy]benzoate was found at -75 ppm, and a quantitative yield thereof was 5%.

Hereinafter, a reaction in Example 19 will be shown.

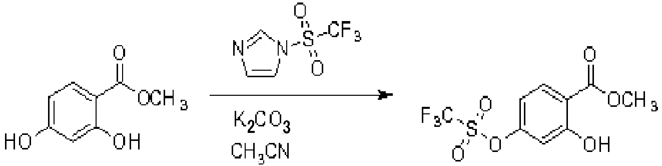

### [Example 20]

After collecting 544 mg (2.00 mmol, 1.0 eq.) of arbutin as a substrate and 10 mL of dimethylformamide as a reaction solvent, a mixture was charged into a 20 mL-sized Schlenk tube equipped with a stirring bar as a reactor. Subsequently, a composition containing both components, that is, 400 mg (2.00 mmol, 1.0 eq.) of 1-trifluoromethanesulfonylimidazole and 14 mg (0.10 mmol, 0.05 eq.) of potassium carbonate, was added to the reactor, and a mixture was stirred at room temperature for 5 hours to obtain a reaction solution. Benzotrifluoride was added to the reaction solution as an internal standard, and when an analysis was performed by ¹⁹F-NMR, a signal derived from 4-trifluoromethanesulfonyloxyphenyl β-D-glucopyranoside was found at -74 ppm, and a quantitative yield thereof was 76%.

Hereinafter, a reaction in Example 20 will be described.

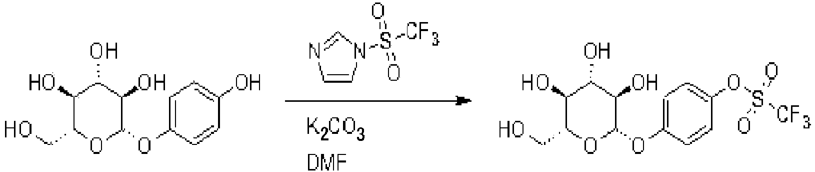

### [Example 21]

After collecting 273 mg (1.00 mmol, 1.0 eq.) of arbutin as a substrate and 5 mL of dimethylformamide as a reaction solvent, a mixture was charged into a 20 mL-sized Schlenk tube equipped with a stirring bar as a reactor. Subsequently, a composition containing both components, that is, 201 mg (1.00 mmol, 1.0 eq.) of 1-trifluoromethanesulfonylimidazole and 19 mg (0.19 mmol, 0.2 eq.) of potassium bicarbonate, was added to the reactor, and a mixture was stirred at room temperature for 22 hours to obtain a reaction solution. Benzotrifluoride was added to the reaction solution as an internal standard, and when an analysis was performed by ¹⁹F-NMR, a signal derived from 4-trifluoromethanesulfonyloxyphenyl β-D-glucopyranoside was found at -74 ppm, and a quantitative yield thereof was 75%.

Hereinafter, a reaction in Example 21 will be described.

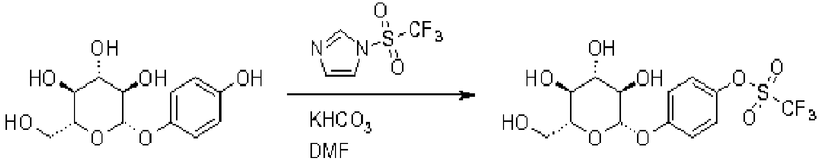

### [Example 22]

After collecting 112 mg (1.03 mmol, 1.0 eq.) of 4-aminophenol as a substrate and 5 mL of acetonitrile as a reaction solvent, a mixture was charged into a 20 mL-sized Schlenk tube equipped with a stirring bar as a reactor. Subsequently, a composition containing both components, that is, 238 mg (1.11 mmol, 1.1 eq.) of 2-methyl-1-trifluoromethanesulfonylimidazole and 27 mg (0.19 mmol, 0.2 eq.) of potassium carbonate, was added to the reactor, and a mixture was stirred at a room temperature for 20 hours to obtain a reaction solution. Benzotrifluoride was added to the reaction solution as an internal standard, and when an analysis was performed by ¹⁹F-NMR, a signal derived from 4-aminophenyl trifluoromethanesulfonate was found at -74 ppm, and a quantitative yield thereof was 80%, but no side reaction was observed. Specifically, no signal derived from "1,1,1-trifluoro-N-(2-hydroxyphenyl)methanesulfonamide" which may correspond to a by-product could be found.

Hereinafter, a reaction in Example 22 will be described.

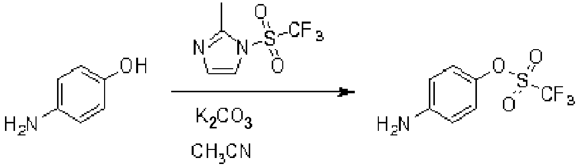

### [Example 23]

### (First Reaction)

After collecting 196 mg (1.00 mmol, 1.0 eq.) of L-tyrosine methyl ester as a substrate and 5 mL of acetonitrile as a reaction solvent, a mixture was charged into a 20 mL-sized Schlenk tube equipped with a stirring bar as a reactor. Subsequently, a composition containing both components, that is, 224 mg (1.12 mmol, 1.1 eq.) of 1-trifluoromethanesulfonylimidazole and 28 mg (0.20 mmol, 0.2 eq.) of potassium carbonate, was added to the reactor, and a mixture was stirred at a room temperature for 2 hours to obtain a reaction solution. The reaction solution was adjusted to pH 1 by adding 5 mL of 1 mol/L hydrochloric acid, and then stirred at the room temperature for 5 minutes. The obtained solution was adjusted to pH 7 by adding 2.5 mL of a 1 mol/L sodium carbonate aqueous solution, and then transferred to a separatory funnel with 20 mL of tert-butyl methyl ether and 20 mL of clean water for layer separation. An obtained organic phase was further washed with 20 mL of clean water and dried with sodium sulfate. The obtained solution was filtered and concentrated under a reduced pressure to obtain 327 mg of methyl (2S)-2-amino-3-(4-trifluoromethanesulfonyloxyphenyl)propanoate in an isolated yield of 100%.

### [Physical Properties]

Methyl (2S)-2-amino-3-(4-trifluoromethanesulfonyloxyphenyl)propanoate
¹H-NMR(400MHz,CDCl₃)δ(ppm):7.30(2H,d,J=9.2Hz),7.22(2H,d,J=9.2Hz),3.74-3.71(4H,m),3.10(1H,dd,J=14.0,5.6Hz),2.90(1H,dd,J=14.0,8.4Hz),1.52(2H,br s).
¹⁹F-NMR(373MHz,CDCl₃)δ(ppm):-73.2(3F,s).

### (Second Reaction)

Into a 20 mL-sized Schlenk tube equipped with a stirring bar as a reactor, 327 mg (1.00 mmol, 1.0 eq.) of methyl (2S)-2-amino-3-(4-trifluoromethanesulfonyloxyphenyl)propanoate obtained in the first reaction, 5 mL of toluene as a reaction solvent, and 0.5 mL of pure water were charged. Nitrogen was bubbled into the obtained solution for 15 minutes, and 204 mg (1.50 mmol, 1.5 eq.) of 4-methylphenylboronic acid, 276 mg (2.00 mmol, 2.0 eq.) of potassium carbonate, and 60 mg (52 µmol, 0.05 eq.) of tetrakis(triphenylphosphine)palladium were collected under a nitrogen gas stream and added to the reactor. The reactor was sealed and stirred at 80°C for 6 hours to obtain a reaction solution. To the reaction solution, 20 mL of ethyl acetate was added, a mixture was washed with 20 mL of clean water, and then an organic phase was dried with sodium sulfate and concentrated under reduced pressure. As an internal standard, 1-methylcyclohexene was added to an obtained residue, and when an analysis was performed by ¹H-NMR, a signal derived from methyl (2S)-2-amino-3-(4'-methyl[1,1'-biphenyl]-4-yl)propanoate was found, and a quantitative yield thereof was 82%.

### [Physical Properties]

### methyl (2S)-2-amino-3-(4'-methyl[1,1'-biphenyl]-4-yl)propanoate

¹H-NMR(400MHz,CDCl₃)8(ppm):7.52(2H,d,J=8.4Hz),7.47(2H,d,J=8.0Hz),7.26-7.23(4H,m),3.79-3.74(4H,m),3.13(1H,dd,J=13.6Hz,4.8Hz),2.89(1H,dd,J=14.0Hz,8.4Hz),2.39(3H,s),1.60(2H,br s).

The first and second reactions in Example 23 are shown below.

First Reaction 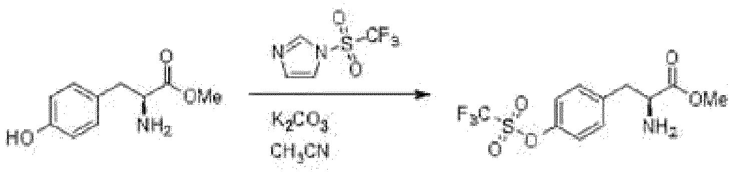
Second Reaction

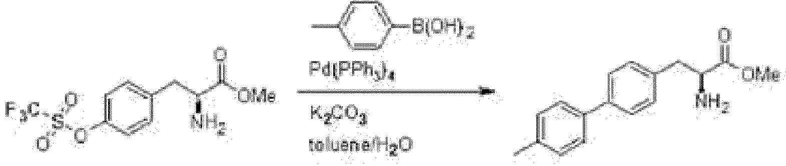

### [Example 24]

### (First Reaction)

After collecting 111 mg (1.02 mmol, 1.0 eq.) of 4-aminophenol as a substrate and 5 mL of acetonitrile as a reaction solvent, a mixture was charged into a 20 mL-sized Schlenk tube equipped with a stirring bar as a reactor. Subsequently, a composition containing both components, that is, 222 mg (1.11 mmol, 1.1 eq.) of 1-trifluoromethanesulfonylimidazole and 28 mg (0.20 mmol, 0.2 eq.) of potassium carbonate, was added to the reactor, and a mixture was stirred at a room temperature for 2 hours to obtain a reaction solution. The reaction solution was adjusted to pH 1 by adding 5 mL of 1 mol/L hydrochloric acid, and then stirred at the room temperature for 5 minutes. The obtained solution was adjusted to pH 7 by adding 2.5 mL of a 1 mol/L sodium carbonate aqueous solution, and then transferred to a separatory funnel with 20 mL of tert-butyl methyl ether and 20 mL of clean water for layer separation. An obtained organic phase was further washed with 20 mL of clean water and dried with sodium sulfate. The obtained solution was filtered and concentrated under reduced pressure to obtain 241 mg of 4-aminophenyl trifluoromethanesulfonate in an isolated yield of 100%.

### [Physical Properties]

4-aminophenyl trifluoromethanesulfonate;
¹H-NMR(400MHz,CDCl₃)8(ppm):7.04(2H,d,J=8.8Hz),6.66(2H,d,J=9.2Hz),3.81(2H,br s).
¹⁹F-NMR(373MHz,CDCl₃)δ(ppm):-73.2(3F,s).

### (Second Reaction)

A 20 mL-sized Schlenk tube equipped with a stirring bar as a reactor was charged with 235 mg (4.04 mmol, 4.0 eq.) of potassium fluoride, 5 mL of tetrahydrofuran as a reaction solvent, and 241 mg (1.00 mmol, 1.0 eq.) of 4-aminophenyl trifluoromethanesulfonate as a substrate, and nitrogen bubbling was performed for 15 minutes. Next, under a nitrogen gas stream, 205 mg (1.51 mmol, 1.5 eq.) of 4-methylphenylboronic acid and 39 mg (50 µmol, 0.05 eq.) of bis(tricyclohexylphosphine)palladium diacetate were collected and added to the reactor. The reactor was sealed and stirred at 60°C for 20 hours to obtain a reaction solution. To the reaction solution, 20 mL of ethyl acetate was added, a mixture was washed with 20 mL of clean water, and then an organic phase was dried with sodium sulfate and concentrated under reduced pressure. As an internal standard, 1,4-bis(trifluoromethyl)benzene was added to an obtained residue, and when an analysis was performed by ¹H-NMR, a signal derived from 4-(4-methylphenyl)aniline was found, and a quantitative yield thereof was 99%.

### [Physical Properties]

4-(4-methylphenyl)aniline
¹H-NMR(400MHz,CDCl₃)δ(ppm):7.43(2H,d,J=8.4Hz),7.39(2H,d,J=8.4Hz),7.20(2H,d,J=8.4Hz),6.75(2H,d,J=8.8Hz),3. 71(2H,br s),2.37(3H,s).

The first and second reactions in Example 24 are shown below.

First Reaction 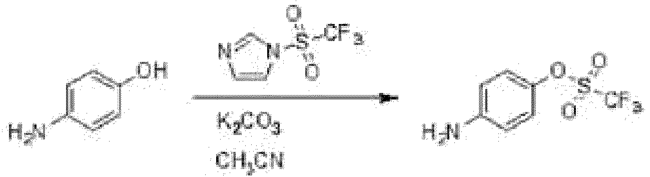
Second Reaction

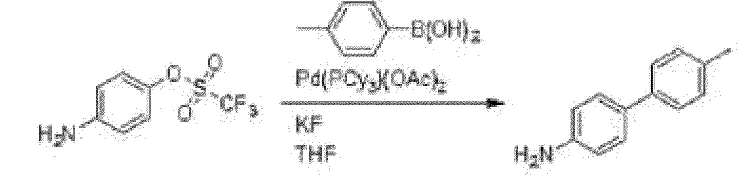

### [Example 25]

### (First Reaction)

After collecting 196 mg (1.01 mmol, 1.0 eq.) of L-tyrosine methyl ester as a substrate and 5 mL of acetonitrile as a reaction solvent, a mixture was charged into a 20 mL-sized Schlenk tube equipped with a stirring bar as a reactor. Subsequently, a composition containing both components, that is, 223 mg (1.11 mmol, 1.1 eq.) of 1-trifluoromethanesulfonylimidazole and 28 mg (0.20 mmol, 0.2 eq.) of potassium carbonate, was added to the reactor, and a mixture was stirred at a room temperature for 2 hours to obtain a reaction solution. The reaction solution was adjusted to pH 1 by adding 5 mL of 1 mol/L hydrochloric acid, and then stirred at the room temperature for 5 minutes. The obtained solution was adjusted to pH 7 by adding 2.5 mL of a 1 mol/L sodium carbonate aqueous solution, and then transferred to a separatory funnel with 20 mL of tert-butyl methyl ether and 20 mL of clean water for layer separation. An obtained organic phase was further washed with 20 mL of clean water and dried with sodium sulfate. The obtained solution was filtered and concentrated under reduced pressure to obtain 328 mg of methyl (2S)-2-amino-3-(4-trifluoromethanesulfonyloxyphenyl)propanoate in an isolated yield of 100%.

### (Second Reaction)

After collecting 328 mg (1.00 mmol, 1.0 eq.) of methyl (2S)-2-amino-3-(4-trifluoromethanesulfonyloxyphenyl)propanoate as a substrate, and 3 mL of dimethylformamide and 3 mL of triethylamine as reaction solvents, a mixture was charged into a 20 mL-sized Schlenk tube equipped with a stirring bar as a reactor. Nitrogen was bubbled into an obtained solution for 15 minutes, and 62 mg (54 µmol, 0.05 eq.) of tetrakis(triphenylphosphine)palladium, 15 mg (77 µmol, 0.08 eq.) of copper iodide, and 179 mg (1.54 mmol, 1.5 eq.) of 4-ethynyltoluene were collected under a nitrogen gas stream and added to the reactor. The reactor was sealed and stirred at 80°C for 20 hours to obtain a reaction solution. To the reaction solution, 30 mL of ethyl acetate was added, a mixture was washed with 30 mL of clean water, and then an organic phase was dried with sodium sulfate and concentrated under reduced pressure. As an internal standard, 1-methylcyclohexene was added to an obtained residue, and when an analysis was performed by ¹H-NMR, a signal derived from methyl (2S)-2-amino-3-{4-[(4-methylphenyl)ethynyl]phenyl}propanoate was found, and a quantitative yield thereof was 91%.

### [Physical Properties]

methyl (2S)-2-amino-3-{4-[(4-methylphenyl)ethynyl]phenyl}propanoate
¹H-NMR(400MHz,CDCl₃)δ(ppm)
:7.46(2H,d,J=8.4Hz),7.42(2H,d,J=8.0Hz),7.18-7.14(4H,m),3.76-3.72(4H,m),3.09(1H,dd,J=13.6,5.6Hz),2.88(1H,dd,J=13.6,8.0Hz),2.37(3H,s),1.50(2H,br s).

The first and second reactions in Example 25 are shown below.

### First Reaction

The same applies to Example 23.

### Second Reaction

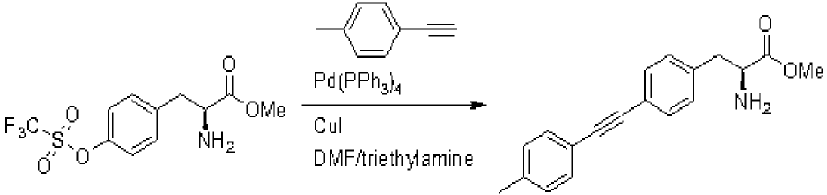

### [Example 26]

### (First Reaction)

After collecting 196 mg (1.00 mmol, 1.0 eq.) of L-tyrosine methyl ester as a substrate and 5 mL of acetonitrile as a reaction solvent, a mixture was charged into a 20 mL-sized Schlenk tube equipped with a stirring bar as a reactor. Subsequently, a composition containing both components, that is, 219 mg (1.09 mmol, 1.1 eq.) of 1-trifluoromethanesulfonylimidazole and 27 mg (0.20 mmol, 0.2 eq.) of potassium carbonate, was added to the reactor, and a mixture was stirred at a room temperature for 2 hours to obtain a reaction solution. The reaction solution was adjusted to pH 1 by adding 5 mL of 1 mol/L hydrochloric acid, and then stirred at the room temperature for 5 minutes. The obtained solution was adjusted to pH 7 by adding 2.5 mL of a 1 mol/L sodium carbonate aqueous solution, and then transferred to a separatory funnel with 20 mL of tert-butyl methyl ether and 20 mL of clean water for layer separation. An obtained organic phase was further washed with 20 mL of clean water and dried with sodium sulfate. The obtained solution was filtered and concentrated under reduced pressure to obtain 320 mg of methyl (2S)-2-amino-3-(4-trifluoromethanesulfonyloxyphenyl)propanoate in an isolated yield of 98%.

### (Second Reaction)

After collecting 320 mg (0.98 mmol, 1.0 eq.) of methyl (2S)-2-amino-3-(4-trifluoromethanesulfonyloxyphenyl)propanoate as a substrate, and 5 mL of dimethylformamide as a reaction solvent, a mixture was charged into a 20 mL-sized Schlenk tube equipped with a stirring bar as a reactor. Nitrogen was bubbled into an obtained solution for 15 minutes, and 516 mg (5.10 mmol, 5.2 eq.) of triethylamine, 239 mg (2.02 mmol, 2.1 eq.) of 4-methylstyrene, and 58 mg (50 µmol, 0.05 eq.) of tetrakis(triphenylphosphine)palladium were added to the reactor under a nitrogen gas stream. The reactor was sealed and stirred at 80°C for 16 hours, and then a temperature was raised to 100°C and stirring was continued for 6 hours to obtain a reaction solution. To the reaction solution, 30 mL of ethyl acetate was added, a mixture was washed with 30 mL of clean water, and then an organic phase was dried with sodium sulfate and concentrated under reduced pressure. As an internal standard, 1,4-bis(trifluoromethyl)benzene was added to an obtained residue, and when an analysis was performed by ¹H-NMR, a signal derived from methyl (2S)-2-amino-3-{4-[(E)-2-(4-methylphenyl)ethenyl]phenyl}propanoate was found, and a quantitative yield thereof was 56%.

### [Physical Properties]

Methyl (2S)-2-amino-3-{4-[(E)-2-(4-methylphenyl)ethenyl]phenyl}propanoate ¹H-NMR(400MHz,CDCl₃)δ(ppm):7.44(2H,d,J=8.4Hz),7.40(2H,d,J=8.0Hz),7.19-7.16(4H,m),7.05-7.04(2H,m),3.76-3.73(4H,m),3.09(1H,dd,J=13.6,5.2Hz),2.87(1H,dd,J=14.0,8.0Hz),2.36(3H,s),1.48(2H, br s).

The first and second reactions in Example 26 are shown below.

### First Reaction

The same applies to Example 23.

### Second Reaction

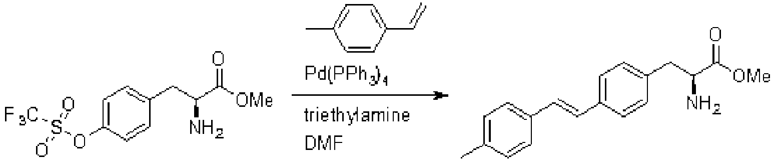

### [Example 27]

### (First Reaction)

After collecting 195 mg (1.00 mmol, 1.0 eq.) of L-tyrosine methyl ester as a substrate and 5 mL of acetonitrile as a reaction solvent, a mixture was charged into a 20 mL-sized Schlenk tube equipped with a stirring bar as a reactor. Subsequently, a composition containing both components, that is, 221 mg (1.10 mmol, 1.1 eq.) of 1-trifluoromethanesulfonylimidazole and 27 mg (0.20 mmol, 0.2 eq.) of potassium carbonate, was added to the reactor, and a mixture was stirred at a room temperature for 2 hours to obtain a reaction solution. The reaction solution was adjusted to pH 1 by adding 5 mL of 1 mol/L hydrochloric acid, and then stirred at the room temperature for 5 minutes. The obtained solution was adjusted to pH 7 by adding 2.5 mL of a 1 mol/L sodium carbonate aqueous solution, and then transferred to a separatory funnel with 20 mL of tert-butyl methyl ether and 20 mL of clean water for layer separation. An obtained organic phase was further washed with 20 mL of clean water and dried with sodium sulfate. The obtained solution was filtered and concentrated under reduced pressure to obtain 327 mg of methyl (2S)-2-amino-3-(4-trifluoromethanesulfonyloxyphenyl)propanoate in an isolated yield of 99%.

### (Second Reaction)

Into a 20 mL-sized Schlenk tube equipped with a stirring bar as a reactor, 327 mg (1.00 mmol, 1.0 eq.) of methyl (2S)-2-amino-3-(4-trifluoromethanesulfonyloxyphenyl)propanoate as a substrate, and 5 mL of dimethyl sulfoxide as a reaction solvent were charged. Nitrogen was bubbled into the obtained solution, and 31 mg (57 µmol, 0.06 eq.) of 1,1'-bis(diphenylphosphino)ferrocene (DPPF) and 11 mg (61 µmol, 0.06 eq.) ofpalladium(II) chloride were charged under a nitrogen gas stream and stirred at a room temperature for 10 minutes. Into an obtained solution, 383 mg (1.51 mmol, 1.5 eq.) of bis(pinacolato)diboron and 309 mg (3.15 mmol, 3.2 eq.) of potassium acetate were charged, and the vessel was then sealed and stirred at 80°C for 16 hours to obtain a reaction solution. To the reaction solution, 30 mL of dichloromethane was added, and a mixture was washed with 30 mL of clean water. An obtained organic phase was dried with sodium sulfate, filtered, and concentrated under reduced pressure. As an internal standard, 1-methylcyclohexene was added to an obtained residue an obtained residue, and when an analysis was performed by ¹H-NMR, a signal derived from methyl (2S)-2-amino-3-[4-(4,4,5,5-tetramethyl-1,3,2-dioxaborolane-2-yl)phenyl]propanoate was found, and a quantitative yield thereof was 87%.

### [Physical Properties]

Methyl (2S)-2-amino-3-[4-(4,4,5,5-tetramethyl-1,3,2-dioxaborolane-2-yl)phenyl]propanoate;
¹H-NMR(400MHz,CDCl₃)δ(ppm):7.75 (2H,d,J=8.0Hz),7.20(2H,d,J=8.0Hz),3.76-3.71(4H,m),3.11(1H,dd,J=13.6,5.6Hz),2.88(1H,dd,J=13.2,8.0Hz),1.73(2H,br s),1.34(12H,s).

### The first and second reactions in Example 27 are shown below.

### First Reaction

The same applies to Example 23.

### Second Reaction

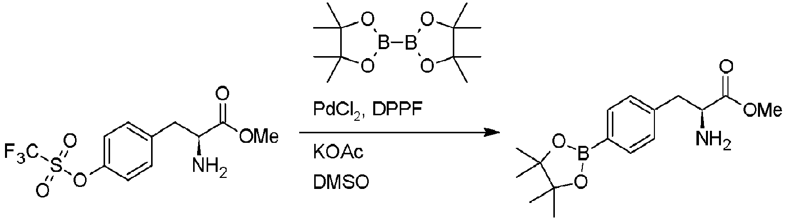

### [Reference Example 1]

After collecting 695 mg (5.03 mmol, 1.0 eq.) of 2-(4-hydroxyphenyl)ethanol as a substrate and 25 mL of dichloromethane as a reaction solvent, a mixture was charged into a 30 mL-sized two-neck eggplant flask equipped with a stirring bar. Subsequently, 521 mg (5.15 mmol, 1.0 eq.) of triethylamine was added to a reactor, and the mixture was stirred while the reactor is cooled with ice. After adding 1.44 g (5.10 mmol, 1.0 eq.) of trifluoromethanesulfonic anhydride dropwise, the reactor was returned to a room temperature and stirred for 2 hours to obtain a reaction solution. Benzotrifluoride was added to the reaction solution as an internal standard, and when an analysis was performed by ¹⁹F-NMR, a signal derived from 4-(2-hydroxyethyl)phenyl trifluoromethanesulfonate was found at -74 ppm, and a quantitative yield thereof was 40%. Further, signals derived from 4-[2-(trifluoromethanesulfonyloxy)ethyl]phenyl trifluoromethanesulfonate as a by-product were observed at -73 ppm and -75 ppm, and a quantitative yield thereof was 29%.

Hereinafter, a reaction in Reference Example 1 will be shown.

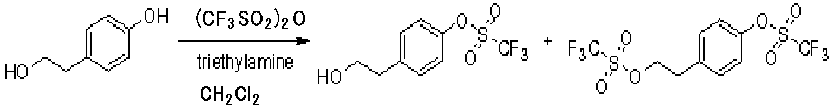

### [Reference Example 2]

After collecting 196 mg (1.00 mmol, 1.0 eq.) of L-tyrosine methyl ester as a substrate and 10 mL of dimethylformamide as a reaction solvent, a mixture was charged into a 20 mL-sized Schlenk tube equipped with a stirring bar as a reactor. Subsequently, 273 mg (1.00 mmol, 1.0 eq.) of 4-nitrophenyl trifluoromethanesulfonate and 278 mg (2.01 mmol, 2.0 eq.) of potassium carbonate were collected and added to the reactor, and a mixture was stirred at room temperature for 2 hours to obtain a reaction solution. Benzotrifluoride was added to the reaction solution as an internal standard, and when an analysis was performed by ¹⁹F-NMR, a signal derived from methyl (2S)-2-amino-3-(4-trifluoromethanesulfonyloxyphenyl)propanoate was found at -74 ppm, a signal derived from methyl (2S)-2-(4-nitroanilino)-3-[4-(trifluoromethanesulfonyloxy)phenyl]propanoate was found at -74 ppm, and quantitative yields thereof were 40% and 27%, respectively.

Hereinafter, a reaction in Reference Example 2 will be shown.

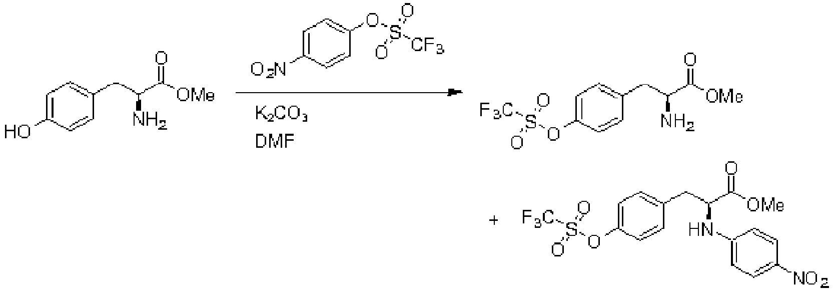

### [Reference Example 3]

After collecting 195 mg (1.00 mmol, 1.0 eq.) of L-tyrosine methyl ester as a substrate and 5 mL of acetonitrile as a reaction solvent, a mixture was charged into a 20 mL-sized Schlenk tube equipped with a stirring bar as a reactor. To the reactor, 106 g (1.05 mmol, 1.0 eq.) of triethylamine was added, and a mixture was stirred while the reactor was cooled to -20°C. After adding 105 µL (1.00 mmol, 1.0 eq.) of trifluoromethanesulfonyl chloride dropwise to the reactor, and a mixture was stirred at -20°C for 2 hours to obtain a reaction solution. Benzotrifluoride was added to the reaction solution as an internal standard, and when an analysis was performed by ¹⁹F-NMR, a signal derived from methyl (2S)-2-amino-3-(4-trifluoromethanesulfonyloxyphenyl)propanoate was observed at -74 ppm, and a quantitative yield thereof was 32%. In addition, two signals derived from by-products were observed at -74 ppm, and quantitative yields thereof were 10% and 8%, respectively.

Hereinafter, a reaction in Reference Example 3 will be shown.

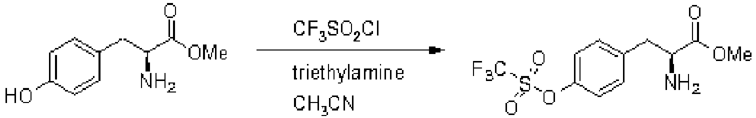

### [Reference Example 4]

After collecting 134 mg (1.00 mmol, 1.0 eq.) of 5-hydroxyindole as a substrate and 4 mL of dichloromethane as a reaction solvent, a mixture was charged into a 20 mL-sized Schlenk tube equipped with a stirring bar as a reactor. To the reactor, 120 mg (1.19 mmol, 1.2 eq.) of triethylamine was added, and a mixture was stirred while the reactor was cooled to -20°C. In 1 mL of dichloromethane, 105 µL (1.00 mmol, 1.0 eq.) of trifluoromethanesulfonyl chloride was dissolved, and the solution was added dropwise to the reactor. A mixture was stirred for 1 hour while the reactor was cooled to -20°C to obtain a reaction solution. Benzotrifluoride was added to the reaction solution as an internal standard, and when an analysis was performed by ¹⁹F-NMR, a signal derived from indol-5-yl trifluoromethanesulfonate was found at -74 ppm, and a quantitative yield thereof was 22%. In addition, one signal derived from a by-product was observed at -73 ppm, and a quantitative yield thereof was 29%.

Hereinafter, a reaction in Reference Example 4 will be shown.

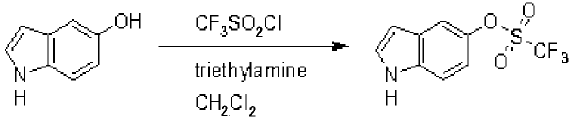

### [Reference Example 5]

As a substrate, 13.8 g (99.9 mmol, 1.4 eq.) of 2-(4-hydroxyphenyl)ethanol was collected and charged into a 300 mL-sized SUS autoclave equipped with a stirring bar as a reactor, and the reactor was then sealed and depressurized with a vacuum pump. After collecting 10.1 g (100 mmol, 1.4 eq.) of triethylamine and 200 mL of acetonitrile as a reaction solvent, a mixture was transferred to the reactor using a cannula. The reactor was cooled with dry ice and charged with 10.6 g (69.4 mmol, 1.0 eq.) of trifluoromethanesulfonyl fluoride from a bomb. The reactor was returned to a room temperature and stirred for 18 hours to obtain a reaction solution. Benzotrifluoride was added to the reaction solution as an internal standard, and when an analysis was performed by ¹⁹F-NMR, a signal derived from 4-(2-hydroxyethyl)phenyl trifluoromethanesulfonate was found at -74 ppm, and a quantitative yield thereof was 33%. In addition, two signals derived from by-products were observed at -74 ppm, and yields thereof were 15% and 11%, respectively.

Hereinafter, a reaction in Reference Example 5 will be shown.

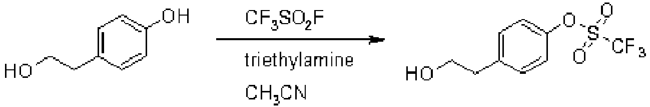

In Examples according to the first aspect, production of the by-products can be prevented and a trifluoromethanesulfonylated compound can be obtained in good yield. The phenolic hydroxyl group can be selectively trifluoromethanesulfonylated.

### (Synthesis Example 4)

### [First Reaction]

After collecting 5.0 g (61 mmol, 1.0 eq. = molar equivalent ratio, the same applies below) of 2-methylimidazole, 19.5 g (116 mmol, 1.9 eq.) of trifluoromethanesulfonyl chloride, 12.9 g (122 mmol, 2.0 eq.) of sodium carbonate, and 61 mL of acetonitrile as a reaction solvent, a mixture was charged into a 200 mL-sized three-neck flask equipped with a stirrer. Thereafter, the mixture was stirred at room temperature (about 25°C, the same applies below) for 22 hours to obtain a first reaction solution. A small amount of the reaction solution was concentrated and analyzed by ¹H-NMR. A signal derived from 2-methylimidazole completely disappeared, and a new signal derived from triflyl-2-methylimidazole was observed, and thus it was found that the first reaction had been completed.

The first reaction solution was filtered through celite (Celite, registered trademark: diatomaceous earth calcined with sodium carbonate, the same applies hereinafter) to remove sodium carbonate, and acetonitrile was removed from a filtrate to obtain 10.2 g of triflyl-2-methylimidazole (also referred to as "1-trifluoromethanesulfonyl-2-methylimidazole").

### [Physical Properties]

Triflyl-2-methylimidazole;
¹H-NMR(400MHz,CD₃CN)δ(ppm):7.42(1H,s),7.05(1H,s),2.56(3H,s)
¹⁹F-NMR(373MHz,CD₃CN)δ(ppm):-76.7(3F,s)

### [Second Reaction]

To 10.2 g of triflyl-2-methylimidazole obtained in the first reaction, 122 mL of methylene chloride and 10.0 g (61 mmol, 1.2 eq.) of methyl trifluoromethanesulfonate were added. Thereafter, a mixture was stirred at a room temperature for 22 hours to obtain a second reaction solution. A small amount of the reaction solution was concentrated and analyzed by ¹H-NMR. A signal derived from triflyl-2-methylimidazole completely disappeared, and a signal derived from triflylimidazolium which is a target product of a second reaction, that is, triflyl-2,3-dimethylimidazolium triflate, was observed, and thus it was found that the second reaction had been completed.

The second reaction solution was concentrated, and 100 ml of tert-butyl methyl ether was added. Thereafter, a mixture was stirred at a room temperature for 22 hours. Precipitated crystals were filtered off under suction and obtained crystals were dried under reduced pressure to obtain 16.7 g of triflyl-2,3-dimethylimidazolium triflate in a yield of 72%.

### [Physical Properties]

Triflyl-2,3-dimethylimidazolium triflate;
¹H-NMR(400MHz,CD₃CN)δ(ppm):7.83(1H,d,J=2.8Hz),7.61(1H,d,J=2.4Hz),3.85(3H,s),2.84(3H,s)
¹⁹F-NMR(373MHz,CD₃CN)δ(ppm):-74.1(3F,s),-79.3(3F,s)

The first and second reactions in Synthesis Example 4 are shown below.

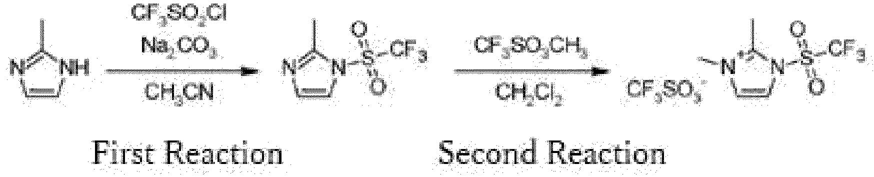

### (Synthesis Example 5)

### [First Reaction]

After collecting 5.0 g (72 mmol, 1.0 eq.) of 1,2,4-triazole, 23.0 g (137 mmol, 1.9 eq.) of trifluoromethanesulfonyl chloride, 15.3 g (144 mmol, 2.0 eq.) of sodium carbonate, and 72 mL of acetonitrile, a mixture was charged into a 200 mL-sized three-neck flask equipped with a stirrer. Thereafter, the mixture was stirred at a room temperature for 3 days to obtain a first reaction solution. A small amount of the reaction solution was concentrated and analyzed by ¹H-NMR. A signal derived from 1,2,4-triazole completely disappeared, and a new signal derived from triflyl-1,2,4-triazole was observed, and thus it was found that a first reaction had been completed.

The first reaction solution was filtered through Celite, and acetonitrile was removed from an obtained filtrate to obtain 12.1 g of triflyl-1,2,4-triazole.

### [Physical Properties]

Triflyl-1,2,4-triazole;
¹H-NMR(400MHz,CD₃CN)δ(ppm):9.05(1H,s),8.38(1H,s)
¹⁹F-NMR(373MHz,CD₃CN)δ(ppm):-75.2(3F,s)

### [Second Reaction]

To 12.1 g of triflyl-1,2,4-triazole obtained in the first reaction, 144 mL of methylene chloride and 11.8 g (72 mmol, 1.2 eq.) of methyl trifluoromethanesulfonate were added. Thereafter, a mixture was stirred at a room temperature for 22 hours to obtain a second reaction solution. A small amount of the reaction solution was concentrated and analyzed by ¹H-NMR. A signal derived from triflyl-1,2,4-triazole completely disappeared, and a signal derived from a triazolium salt which is a target product of a second reaction, that is, triflyl-4-methyl-1,2,4-triazolium triflate, was observed, and thus it was found that the second reaction had been completed.

The second reaction solution was concentrated, and 100 ml of tert-butyl methyl ether was added. Thereafter, a mixture was stirred at a room temperature for 22 hours. Precipitated crystals were filtered off under suction and obtained crystals were dried under reduced pressure to obtain 16.5 g of triflyl-4-methyl-1,2,4-triazolium triflate in a yield of 63%.

### [Physical Properties]

Triflyl-4-methyl-1,2,4-triazolium triflate;
¹H-NMR(400MHz,CD₃CN)δ(ppm):10.40(1H,s),9.00(1H,s),4.07(3H,s)
¹⁹F-NMR(373MHz,CD₃CN)δ(ppm):-71.5(3F,s),-79.3(3F,s)

The first and second reactions in Synthesis Example 5 are shown below.

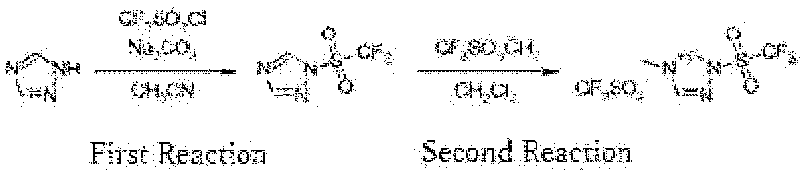

### (Synthesis Example 6)

### [First Reaction]

After collecting 5.0 g (73 mmol, 1.0 eq.) of imidazole, 23.5 g (139 mmol, 1.9 eq.) of trifluoromethanesulfonyl chloride, 15.6 g (122 mmol, 2.0 eq.) of sodium carbonate, and 73 mL of acetonitrile as a reaction solvent, a mixture was charged into a 200 mL-sized three-neck flask equipped with a stirrer. Thereafter, a mixture was stirred at a room temperature for 16 hours to obtain a first reaction solution. A small amount of the reaction solution was concentrated and analyzed by ¹H-NMR. A signal derived from imidazole completely disappeared, and a new signal derived from triflylimidazole was observed, and thus it was found that the first reaction had been completed.

The first reaction solution was filtered through Celite to remove sodium carbonate, and acetonitrile was removed from a filtrate to obtain 9.7 g of triflylimidazole (also referred to as "1-trifluoromethanesulfonylimidazole").

### [Physical Properties]

Triflylimidazole;
¹H-NMR(400MHz,CD₃CN)δ(ppm):8.15(1H,s),7.56(1H,s),7.29(1H,s)
¹⁹F-NMR(373MHz,CD₃CN)δ(ppm):-77.3(3F,s)

### [Second Reaction]

To 9.7 g of triflylimidazole obtained in the first reaction, 50 mL of methylene chloride, and 7.9 g (48 mmol, 1.0 eq.) of methyl trifluoromethanesulfonate were added. Thereafter, a mixture was stirred at a room temperature for 14 hours to obtain a second reaction solution. A small amount of the reaction solution was concentrated and analyzed by ¹H-NMR. A signal derived from triflylimidazole completely disappeared, and a signal derived from triflylimidazolium which is a target product of a second reaction, that is, triflyl-3-methylimidazolium triflate, was observed, and thus it was found that the second reaction had been completed.

The second reaction solution was concentrated, and 100 ml of tert-butyl methyl ether was added. Thereafter, a mixture was stirred at a room temperature for 15 hours. Precipitated crystals were filtered off under suction and obtained crystals were dried under reduced pressure to obtain 13.6 g of triflyl-3-methylimidazolium triflate in a yield of 50%.

### [Physical Properties]

Triflyl-3-methylimidazolium triflate;
¹H-NMR(400MHz,CD₃CN)δ(ppm):9.45(1H,s),7.97(1H,m),7.74(1H,m),4.01(3H,s)
¹⁹F-NMR(373MHz,CD₃CN)δ(ppm):-73.9(3F,s),-79.3(3F,s)

The first and second reactions in Synthesis Example 6 are shown below.

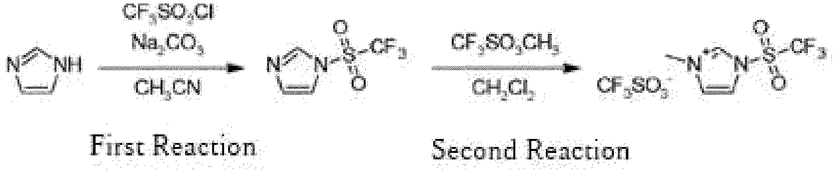

### (Synthesis Example 7)

After collecting 0.81 g (3.8 mmol, 1.0 eq.) of triflyl-2-methylimidazole obtained in the same manner as in the first reaction in Synthesis Example 4 and 4 mL of methylene chloride, a mixture was charged into a 20 mL-sized Schlenk tube equipped with a stirring bar as a reactor. Further, 0.68 g (3.8 mmol, 1.0 eq.) of ethyl trifluoromethanesulfonate was added, and a mixture was stirred at 60°C for 2 hours. A small amount of a reaction solution was concentrated and analyzed by ¹H-NMR. A signal derived from triflyl-2-methylimidazole completely disappeared, and a signal derived from triflylimidazolium which is a target product, that is, triflyl-3-ethyl-2-methylimidazolium triflate, was observed, and thus it was found that a second reaction had been completed.

To the reaction solution, 20 mL of tert-butyl methyl ether was added, and a mixture was stirred at room temperature for 1 hour. Precipitated crystals were filtered off under suction and obtained crystals were dried under reduced pressure to obtain 1.3 g of triflyl-3-ethyl-2-methylimidazolium triflate in a yield of 88%.

### [Physical Properties]

Triflyl-3-ethyl-2-methylimidazolium triflate;
¹H-NMR(400MHz,CD₃CN)δ(ppm):7.86(1H,d,J=2.8Hz),7.69(1H,d,J=2.4Hz),4.25(2H,q,J=7.6Hz),2.86(3H,s),1.47(3H,t, J=7.6Hz)
¹⁹F-NMR(373MHz,CD₃CN)δ(ppm):-74.1(3F,s),-79.3(3F,s)

Hereinafter, a reaction in Synthesis Example 7 will be shown.

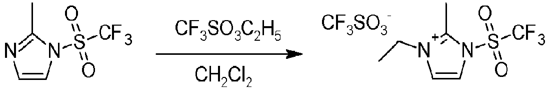

### [Example 28]

After collecting 93 mg (1.0 mmol, 1.0 eq.) of aniline as a substrate and 5 mL of acetonitrile as a reaction solvent, a mixture was charged into a 20 mL-sized Schlenk tube equipped with a stirring bar as a reactor. While the Schlenk tube was cooled in an ice bath, a composition containing both components, that is, 0.95 g (2.5 mmol, 2.5 eq.) of triflyl-2,3-dimethylimidazolium triflate and 51 mg (0.5 mmol, 0.5 eq.) of triethylamine, was further added to the Schlenk tube. After the ice bath was removed, a mixture was stirred at a room temperature for 2 hours to obtain a reaction solution. Benzotrifluoride was added to the reaction solution as an internal standard, and when an analysis was performed by ¹⁹F-NMR, a signal derived from N-phenyl triflimide was found at -71 ppm, and a quantitative yield thereof was 98%.

Hereinafter, a reaction in Example 28 will be described.

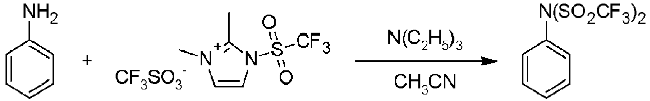

### [Example 29]

After collecting 73 mg (1.0 mmol, 1.0 eq.) of butylamine as a substrate and 5 mL of acetonitrile, a mixture was charged into a 20 mL-sized Schlenk tube equipped with a stirring bar as a reactor. While the Schlenk tube was cooled in an ice bath, a composition containing both components, that is, 0.95 g (2.5 mmol, 2.5 eq.) of triflyl-2,3-dimethylimidazolium triflate and 51 mg (0.5 mmol, 0.5 eq.) of triethylamine, was further added to the Schlenk tube. After removing the ice bath, a mixture was stirred at a room temperature for 24 hours to obtain a reaction solution. Benzotrifluoride was added to the reaction solution as an internal standard, and when an analysis was performed by ¹⁹F-NMR, a signal derived from N-butyl triflimide was found at -73 ppm, a signal derived from N-butyltriflamide was found at -78 ppm, and quantitative yields thereof were 94% for N-butyltriflimide and 5% for N-butyltriflamide.

Hereinafter, a reaction in Example 29 will be shown.

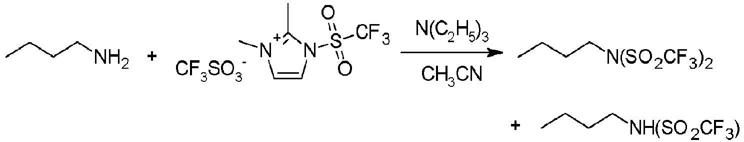

### [Example 30]

After collecting 0.12 g (1.0 mmol, 1.0 eq.) of 1-phenylethylamine as a substrate and 5 mL of acetonitrile, a mixture was charged into a 20 mL-sized Schlenk tube equipped with a stirring bar as a reactor. While the Schlenk tube was cooled in an ice bath, a composition containing both components, that is, 0.95 g (2.5 mmol, 2.5 eq.) of triflyl-2,3-dimethylimidazolium triflate and 51 mg (0.5 mmol, 0.5 eq.) of triethylamine, was further added to the Schlenk tube. After removing the ice bath, a mixture was stirred at a room temperature for 2 hours to obtain a reaction solution. Benzotrifluoride was added to the reaction solution as an internal standard, and when an analysis was performed by ¹⁹F-NMR, a signal derived from N-(1-phenylethyl)triflamide was found at -79 ppm, and a quantitative yield thereof was 100%.

Hereinafter, a reaction in Example 30 will be shown.

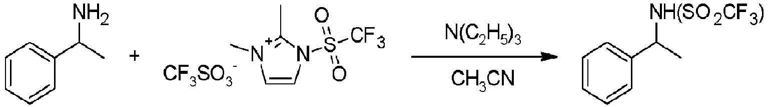

### [Example 31]

After collecting 0.15 g (1.0 mmol, 1.0 eq.) of 6-isopropyl-3-methylphenol as a substrate and 5 mL of acetonitrile, a mixture was charged into a 20 mL-sized Schlenk tube equipped with a stirring bar as a reactor. While the Schlenk tube was cooled in an ice bath, a composition containing both components, that is, 0.57 g (1.5 mmol, 1.5 eq.) of triflyl-2,3-dimethylimidazolium triflate and 51 mg (0.5 mmol, 0.5 eq.) of triethylamine, was further added to the Schlenk tube. After removing the ice bath, a mixture was stirred at a room temperature for 2 hours to obtain a reaction solution. Benzotrifluoride was added to the reaction solution as an internal standard, and when an analysis was performed by ¹⁹F-NMR, a signal derived from 2-isopropyl-5-methylphenyl triflate was found at -75 ppm, and a quantitative yield thereof was 99%.

Hereinafter, a reaction in Example 31 will be shown.

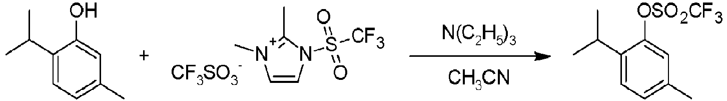

### [Example 32]

After collecting 0.31 g (3.0 mmol, 1.0 eq.) of methyl lactate as a substrate and 15 mL of acetonitrile, a mixture was charged into a 30 mL-sized two-neck flask equipped with a stirring bar. While the two-neck flask was cooled in an ice bath, a composition containing both components, that is, 1.70 g (4.5 mmol, 1.5 eq.) of triflyl-2,3-dimethylimidazolium triflate and 91 mg (0.9 mmol, 0.3 eq.) of triethylamine, was further added to the two-neck flask. After removing the ice bath, a mixture was stirred at a room temperature for 3 hours to obtain a reaction solution. Benzotrifluoride was added to the reaction solution as an internal standard, and when an analysis was performed by ¹⁹F-NMR, a signal derived from methyl 2-trifluoromethanesulfonyloxypropionate was found at -76 ppm, and a quantitative yield thereof was 71%.

Hereinafter, a reaction in Example 32 will be shown.

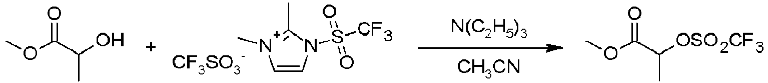

### [Example 33]

After collecting 0.33 g (2.5 mmol, 1.0 eq.) of ethyl acetoacetate as a substrate, 12.5 mL of tetrahydrofuran as a reaction solvent, and 0.10 g (2.5 mmol, 1.0 eq.) of sodium hydride (60% by mass oil dispersion), a mixture was charged into a 30 mL-sized two-neck flask equipped with a stirring bar, and then stirred at room temperature for 10 minutes. Next, 1.13 g (3.0 mmol, 1.2 eq.) of triflyl-2,3-dimethylimidazolium triflate was added to the two-neck flask, and a mixture was further stirred at room temperature for 1 hour to obtain a reaction solution. Benzotrifluoride was added to the reaction solution as an internal standard, and when an analysis was performed by ¹⁹F-NMR, a signal derived from ethyl (Z)-3-trifluoromethanesulfonyloxy-2-butenoate was found at -76 ppm, and a quantitative yield thereof was 87%.

Hereinafter, a reaction in Example 33 will be shown.

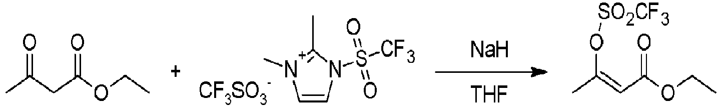

### [Example 34]

After collecting 93 mg (1.0 mmol, 1.0 eq.) of aniline as a substrate and 5 mL of acetonitrile, a mixture was charged into a 20 mL-sized Schlenk tube equipped with a stirring bar as a reactor. While the Schlenk tube was cooled in an ice bath, a composition containing both components, that is, 0.91 g (2.5 mmol, 2.5 eq.) of triflyl-3-methylimidazolium triflate and 51 mg (0.5 mmol, 0.5 eq.) of triethylamine, was further added to the Schlenk tube. After removing the ice bath, a mixture was stirred at a room temperature for 1 hour to obtain a reaction solution. Benzotrifluoride was added to the reaction solution as an internal standard, and when an analysis was performed by ¹⁹F-NMR, a signal derived from N-phenyl triflimide was found at -71 ppm, and a quantitative yield thereof was 93%.

Hereinafter, a reaction in Example 34 will be shown.

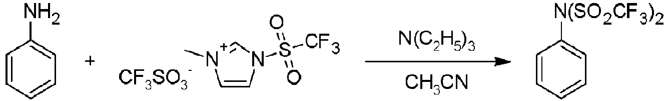

### [Example 35]

After collecting 47 mg (0.5 mmol, 1.0 eq.) of aniline as a substrate and 2.5 mL of acetonitrile, a mixture was charged into a 20 mL-sized Schlenk tube equipped with a stirring bar as a reactor. While the Schlenk tube was cooled in an ice bath, a composition containing both components, that is, 0.49 g (1.3 mmol, 2.5 eq.) of triflyl-3-ethyl-2-methylimidazolium triflate synthesized by the method described in Example 4 and 25 mg (0.25 mmol, 0.5 eq.) of triethylamine, was further added to the Schlenk tube. After the ice bath was removed, a mixture was stirred at a room temperature for 1 hour to obtain a reaction solution. Benzotrifluoride was added to the reaction solution as an internal standard, and when an analysis was performed by ¹⁹F-NMR, a signal derived from N-phenyl triflimide was found at -71 ppm, and a quantitative yield thereof was 92%.

Hereinafter, a reaction in Example 35 will be shown.

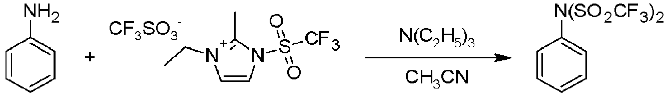

As described above, when trifluoromethanesulfonylation was carried out using the triflylimidazolium salts or triazolium salts obtained in Synthesis Examples 4 to 7, excellent performance was shown as trifluoromethanesulfonylating agents for a plurality of types of substrates.

### INDUSTRIAL APPLICABILITY

A trifluoromethanesulfonylating agent composition of the present disclosure can be utilized as a trifluoromethanesulfonylating agent composition under industrially feasible conditions in synthesis of active pharmaceutical ingredients or pharmaceutical intermediates.

Although the present disclosure has been described in detail and with reference to specific examples, it will be apparent to those skilled in the art that various changes and modifications can be made without departing from the spirit and scope of the present disclosure.

The present application is based on a Japanese Patent Application (No. 2022-076302) filed on May 2, 2022, the contents of which are incorporated herein by reference.

## Claims

1. A trifluoromethanesulfonylating agent composition comprising:
a compound represented by the following Formula (1) or (11): 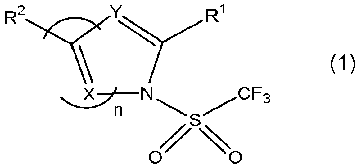 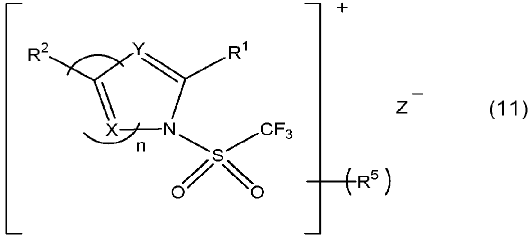
wherein, in Formulae (1) and (11), R¹ is a hydrogen atom, a linear alkyl group having 1 to 6 carbon atoms, or a branched alkyl group having 3 to 6 carbon atoms,
R² is a hydrogen atom, a linear aliphatic hydrocarbon group having 1 to 6 carbon atoms, a branched aliphatic hydrocarbon group having 3 to 6 carbon atoms, a nitro group, an aromatic hydrocarbon group having 6 to 14 carbon atoms, or an aromatic heterocyclic group having 6 to 14 carbon atoms,
X is a nitrogen atom or C(R³),
Y is a nitrogen atom or C(R⁴),
R³ is a hydrogen atom, a linear aliphatic hydrocarbon group having 1 to 6 carbon atoms, a branched aliphatic hydrocarbon group having 3 to 6 carbon atoms, an aromatic hydrocarbon group having 6 to 14 carbon atoms, or an aromatic heterocyclic group having 6 to 14 carbon atoms,
R⁴ is a hydrogen atom, a linear aliphatic hydrocarbon group having 1 to 6 carbon atoms, a branched aliphatic hydrocarbon group having 3 to 6 carbon atoms, an aromatic hydrocarbon group having 6 to 14 carbon atoms, or an aromatic heterocyclic group having 6 to 14 carbon atoms,
R² and R³ may be bonded to form a ring, and R² and R⁴ may be bonded to form a ring, and
n is an integer of 1 to 3;
when there are a plurality of R², R² may be the same or different, and when there are a plurality of R³, R³ may be the same or different; and
in Formula (11), R⁵ is a hydrogen atom, a linear alkyl group having 1 to 6 carbon atoms, or a branched alkyl group having 3 to 6 carbon atoms, and
Z⁻ is an anion.

2. The trifluoromethanesulfonylating agent composition according to claim 1, further comprising:
a compound represented by Formula (1); and
any base selected from the group consisting of an aliphatic organic base, an organic base having a heterocyclic group, and an inorganic base.

3. The trifluoromethanesulfonylating agent composition according to claim 2,
wherein, in Formula (1), X is C(R³), and R¹, R², and R³ each independently represent a hydrogen atom, a methyl group, an ethyl group, an n-propyl group, or an isopropyl group.

4. The trifluoromethanesulfonylating agent composition according to claim 2,
wherein the organic base is selected from the group consisting of a secondary amine, a tertiary amine, an alkoxide, and an organic base having a heterocyclic group having a nitrogen atom and 4 or more carbon atoms.

5. The trifluoromethanesulfonylating agent composition according to claim 2,
wherein the inorganic base is selected from the group consisting of a potassium salt and a sodium salt.

6. The trifluoromethanesulfonylating agent composition according to claim 2,
wherein the base is any one selected from the group consisting of sodium carbonate, potassium carbonate, sodium bicarbonate, potassium bicarbonate, lithium methoxide, lithium ethoxide, lithium tert-butoxide, sodium methoxide, sodium ethoxide, sodium tert-butoxide, potassium methoxide, potassium ethoxide, potassium tert-butoxide, triethylamine, tri-n-propylamine, tributylamine, diisopropylethylamine, N,N-diethylcyclohexylamine, 1,8-diazabicyclo[5.4.0]undecene, and 1,5-diazabicyclo[4.3.0]nonene.

7. A method for producing a trifluoromethanesulfonyloxy compound, comprising:
reacting the trifluoromethanesulfonylating agent composition according to any one of claims 2 to 6 with a hydroxylated aromatic compound represented by Formula (2):
**Ar-OH** (2)
wherein, in Formula (2), Ar represents an aromatic ring group or a substituted aromatic ring group.

8. The method for producing a trifluoromethanesulfonyloxy compound according to claim 7,
wherein, in Formula (2), Ar represents an aromatic ring group, and the aromatic ring group is an aromatic heterocyclic group.

9. The method for producing a trifluoromethanesulfonyloxy compound according to claim 7,
wherein, in Formula (2), Ar represents a substituted aromatic ring group, and a substituent of the substituted aromatic ring group is a lower alkyl group, a lower alkoxycarbonyl lower alkyl group, a β-D-glucopyranoside group, an amino group, a lower alkylamino group, or a hydroxyl group.

10. The method for producing a trifluoromethanesulfonyloxy compound according to claim 7,
wherein the reaction is performed at a reaction temperature of 150°C or lower.

11. The method for producing a trifluoromethanesulfonyloxy compound according to claim 7,
wherein after completion of the reaction, a reaction solution is post-treated with an acidic aqueous solution.

12. The trifluoromethanesulfonylating agent composition according to claim 1, wherein
a compound represented by Formula (11) is a compound represented by the following Formula (11a): 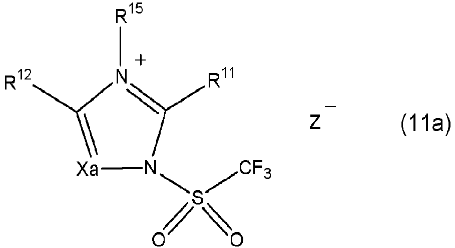
wherein, in Formula (11a), R¹¹, R¹², and R¹⁵ are a hydrogen atom, a linear alkyl group having 1 to 6 carbon atoms, or a branched alkyl group having 3 to 6 carbon atoms,
Xa is a nitrogen atom or C(R¹³),
R¹³ is a hydrogen atom, a linear alkyl group having 1 to 6 carbon atoms, or a branched alkyl group having 3 to 6 carbon atoms, and
R¹² and R¹³ may be bonded to form a ring; and
Z⁻ is an anion.

13. The trifluoromethanesulfonylating agent composition according to claim 12,
wherein Z⁻ in Formula (11a) is a trifluoromethanesulfonate anion, a fluoromethanesulfonate anion, a methanesulfonate anion, or a sulfonate anion.

14. The trifluoromethanesulfonylating agent composition according to claim 12,
wherein Xa in Formula (11a) is C(R¹³).

15. The trifluoromethanesulfonylating agent composition according to claim 12,
wherein, in Formula (11a), Xa is C(R¹³), and R¹¹, R¹², R¹³, and R^{1S} are each independently a hydrogen atom, a methyl group, an ethyl group, an n-propyl group, or an isopropyl group.

16. A method for producing a trifluoromethanesulfonyl compound, comprising:
reacting the trifluoromethanesulfonylating agent composition according to any one of claims 12 to 15 with at least one substrate selected from the group consisting of a compound having a phenolic hydroxyl group, a compound having an alcoholic hydroxyl group, a ketone, a primary amine, and a secondary amine.
